Europäisches Patentamt

European Patent Office

Office européen des brevets

(11) Publication number: **0 127 839 B1**

(12) **EUROPEAN PATENT SPECIFICATION**

(45) Date of publication of patent specification: **15.07.92**

(21) Application number: **84105841.5**

(22) Date of filing: **22.05.84**

(51) Int. Cl.⁵: **C12N 15/00**, C12P 21/02, C12N 15/62, C12N 15/86, C07K 15/00

(54) **Method for producing a recombinant baculovirus expression vector.**

(30) Priority: **27.05.83 US 498858**
**16.05.84 US 609697**

(43) Date of publication of application:
**12.12.84 Bulletin 84/50**

(45) Publication of the grant of the patent:
**15.07.92 Bulletin 92/29**

(84) Designated Contracting States:
**AT BE CH DE FR GB IT LI LU NL SE**

(56) References cited:

**JOURNAL OF VIROLOGY, vol. 44, no. 3, December 1982, Washington DC. M.J. ADANG et al. "Molecular Cloning of DNA Complementary to mRNA of the Baculovirus Autographa californica Nuclear Polyhedrosis Virus: Location and Gene Products of RNA Transcripts Found Late in Infection" pages 782-793**

(73) Proprietor: **THE TEXAS A&M UNIVERSITY SYSTEM**
**Mail Stop 1169 Room 216 Teague Building**
**College Station Texas 77843-1120(US)**

(72) Inventor: **Smith, Gale E.**
**2106 Pantera**
**Bryan Texas 77801(US)**
Inventor: **Summers, Max D.**
**1908 Streamside Way**
**Bryan Texas 77801(US)**

(74) Representative: **Glawe, Delfs, Moll & Partner**
**Patentanwälte**
**Postfach 26 01 62 Liebherrstrasse 20**
**W-8000 München 26(DE)**

Rank Xerox (UK) Business Services

EP 0 127 839 B1

JOURNAL OF VIROLOGY, vol. 45, no. 1, January 1983, Washington DC. G.E. SMITH et al. "Physical Analysis of Autographa californica Nuclear Polyhedrosis Virus Transcripts for Polyhedrin and 10,000-Molecular-Weight Protein" pages 215-225

JOURNAL OF VIROLOGY, vol. 30, no. 3, June 1979, Washington DC. G.E. SMITH et al. "Restriction Maps of Five Autographa californica MNPV Variants, Trichoplusia ni MNPV, and Galleria mellonella MNPV DNAs with Endonucleases SmaI, KpnI, BamHI, SacI, XhoI, and EcoRI" pages 828-838

JOURNAL OF VIROLOGY, vol. 33, no. 1, January 1980, Washington DC. G.E. SMITH et al. "Restriction Map of Rachiplusia ou and Rachiplusia ou - Autographa californica Baculovirus Recombinants" pages 311-319

2

## Description

This invention relates to a method for producing a recombinant viral expression vector. More particularly, this invention relates to a method for incorporating a selected gene coupled with a baculovirus promoter into a baculovirus genome to produce a recombinant baculovirus expression vector capable of expression of the selected gene in an insect cell.

Recent advances in recombinant DNA technology have facilitated the isolation of specific genes or parts thereof and their transfer to bacteria, yeast, plant, or animal cells and to the viruses that infect these organisms. The transferred gene material [or modified gene(s)] is replicated and propagated as the transformed cells or viruses replicate. As a result, the transformed cells take on the capacity to produce the product for which the transferred gene sequences encode.

The transfer and expression of genes, or portions thereof, between viruses, eukaryotes and prokaryotes is possible because the DNA of all living organisms is chemically similar in that it is composed of the same four nucleotides. The basic differences reside in the sequences in which the nucleotides appear in the genome of the organism. Specific nucleotide sequences, arranged in codons (nucleotide triplets), code for specific amino acid sequences. However, the coding relationship between an amino acid sequence and a DNA nucleotide sequence is essentially the same for all organisms.

Genomic DNA is organized into protein encoding sequences (i.e., "structural genes") and control regions (the DNA sequences that control transcriptional initiation are usually referred to as the "promoter") that mediate expression of the structural gene. In general, the enzyme RNA polymerase is activated by the promoter such that as it travels along the structural gene, it transcribes encoded information into a messenger ribonucleic acid (mRNA). The mRNA contains recognition sequences, signals for ribosome binding, and signals for translational start and stop. Recent advances in the genetic analysis of the role of important transcriptional signals in the promoter regions of genes (which are usually described as the 5' flanking region of genes) have facilitated the ability to selectively remove or alter DNA sequences to study their function and role in expression, and to remove certain of these sequences to study their function in heterologous biological systems such as a recombinant DNA host-vector system.

Eukaryotic promoters are usually characterized by two conserved sequences of nucleotides whose locations and structural similarity to prokaryotic promoter sequences (Breathnach & Chambon, 50 Ann. Rev. Biochem. 349-383 (1981)) suggest involvement in the promotion of transcription. The first is a sequence rich in the nucleic acids adenine and thymine (the Goldberg-Hogness, "TATA," or "ATA" box) which is located 20-30 base pairs upstream from the RNA initiation site (the cap site which is the transcriptional start site for the mRNA) and is characterized by a consensus sequence (5'-TATAA-ATA-3'). The second region is the CCAAT box (Efstratadis, et al., 21 Cell 653-668 (1980)), which is located 70-90 base pairs up-stream from the cap site of some genes and has the canonical sequence 5'-GG(C/T)CAATCT-3' (Benoist, et al., 8 Nucleic Acids Res. 127-142 (1980)). These sequences may be removed and modified by the use of restriction endonuclease enzymes and cloning to produce recombinant DNA molecules and the controlled removal or alteration of the cloned nucleotide sequences by in vitro or site-specific mutagenesis. Restriction endonucleases are hydrolytic enzymes capable of catalyzing the site-specific cleavage of DNA molecules. The site of restriction enzyme activity is determined by the presence of a specific nucleotide sequence and is termed the recognition site for a given restriction endonuclease. Many restriction enzymes have been isolated and classified according to their recognition site. Some restriction endonucleases hydrolyze the phospho-diester bonds on both DNA strands at the same point to produce blunt ends, while others hydrolyze bonds which are separated by a few nucleotides from each other to produce free single-stranded regions at the end of each DNA molecule. These single-stranded ends are self-complementary and may be used to rejoin the hydrolyzed DNA or another or heterologous DNA sequences with the same complementary single-stranded sequences.

Restriction sites are relatively rare. However the general use of restriction endonucleases has been greatly improved by the availability of chemically synthesized double-stranded oligonucleotides containing the desired restriction site sequence. Virtually any naturally occurring, cloned, genetically altered or chemically synthesized segment of DNA can be coupled to any other segment by attaching an oligonucleotide containing the appropriate recognition sites to the ends of the DNA molecule. Subjecting this product to the hydrolytic action of the appropriate restriction endonuclease produces the requisite complementary ends for coupling the DNA molecules.

Recognition sites for specific restriction enzymes are usually randomly distributed. Therefore, cleavage by a restriction enzyme may occur between adjacent codons, within a codon or at some random site in the gene. While there are many possible variations on this scheme, it is important to note that techniques are available for inserting DNA sequences in the proper location and orientation with respect to a promoter region to allow expression of those sequences.

Potentially, any DNA sequence can thus be cloned by inserting a foreign DNA sequence into a cloning vehicle or vector molecule to construct an artificial recombinant molecule or composite sometimes called a chimera or hybrid gene. For most purposes, the cloning vehicle utilized is a duplex extrachromosomal DNA sequence comprising an intact replicon such that the recombinant molecule can be replicated when placed into bacteria, yeast, plant or animal cells by transformation. Cloning vehicles commonly in use are derived from viruses and plasmids associated with bacteria, yeast, plant and animal cells.

Recent advances in biochemistry and recombinant DNA technology have led to the construction of cloning vehicles or vectors containing "heterologous" DNA. The term heterologous" refers to DNA that codes for polypeptides ordinarily not produced by the cell. The heterologous DNA can be incorporated into the genome of the cell or maintained in the transformed cell on self-replicating plasmid or virus cloning vehicles. These transformed cell populations provide a renewable source of the heterologous DNA for further manipulations, modifications and transfer to other vectors. Certain viral vectors carrying foreign gene(s) will replicate in and lyse the transformed cells. During replication, the foreign gene(s) may or may not be expressed in that particular cell type. The replicated virus can be isolated and used to infect additional cells and thus provide a renewable source of the recombinant for further use.

Once the gene or desirable portions thereof have been cloned and or biochemically modified in the desired manner or other biochemically modified or genomic genes have been inserted in such a manner as to facilitate their expression, they are then transferred to an expression vector. Because of the nature of the genetic code, the cloned or hybrid gene or portions thereof will direct the production of the amino acid sequences for which it codes. The general techniques for constructing expression vectors with cloned genes located in the proper relationship to promoter regions are described by B. Polisky, et al., 73 Proc. Natl. Acad. Sci. U.S.A. 3900 (1976), K. Itakura, et al., 198 Science 1056-1063 (1977), L. Villa-Komaroff, et al., 75 Proc. Natl. Acad. Sci. U.S.A. 3727-3731 (1978) and others.

The term "expression" may be characterized in the following manner. Even in relatively simple prokaryotic organisms, the cell is capable of synthesizing many proteins. At any given time, many proteins which the cell is capable of synthesizing are not being synthesized. When a particular polypeptide, coded for by a given gene, is being synthesized by the cell, that gene is said to be expressed. In order to be expressed, the DNA sequence coding for that particular polypeptide must be properly located with respect to the control region of the gene. The function of the control region is to permit the expression of the gene under its control to be responsive to the changing needs of the cell at any given moment.

As used throughout this specification, the following definitions apply:

A cloning vehicle is an extra-chromosomal length of duplex DNA comprising an intact replicon that can be replicated within cells or an organism by transformation. Generally, cloning vehicles are derived from viruses or plasmids, and most commonly take the form of circular DNA.

The term gene refers to those DNA sequences which are responsible for the transmission and synthesis of a single protein chain.

The term infection refers to the invasion by pathogenic viral agents of cells where conditions are favorable for their replication and growth.

The term transfection refers to a technique for infecting cells with purified nucleic acids by precipitation of DNAs and uptake into cells upon addition of calcium chloride to solutions of DNA containing phosphate or other appropriate agents such as dextran sulfate.

A number of host-vector systems utilizing the above-described general scheme and techniques have been developed for use in the commercial or experimental synthesis of proteins by genetically modified organisms. Many of these host-vector systems are prokaryotic host-vector systems, such as that described in U.S. Patent No. 4,338,397 to Gilbert, et al. Additionally, systems have been utilized which employ yeast as a vector such as the system employed for hepatitis B surface antigen synthesis as described by A. Miyanohara, et al., 80 Proc. Natl. Acad. Sci. U.S.A. 1 (1983), and the system for human interferon synthesis within yeast described by Pitzeman, et al., 219 Science 620 (1983).

The value of utilizing prokaryotic hostvector systems for the synthesis of desirable proteins is diminished by certain limitations inherent in such systems. For instance, the mRNA transcript or protein product of such systems may be unstable in the prokaryote. In addition, before a protein will be synthesized within a prokaryotic cell, the DNA sequence introduced into the microorganism must be free of intervening DNA sequences, nonsense sequences, and initial or terminal sequences which encode for polypeptide sequences which do not comprise the active eukaryotic protein. Further, some eukaryotic proteins require modification after synthesis (i.e., glycosylation) to become biologically active, and prokaryotic cells are generally incapable of such modifcations.

An additional limitation associated with yeast or prokaryotic host-vector systems includes the difficulties associated with the recovery of gene products synthesized from within the cell. U.S. Patent No. 4,336,336 to Silhavy, et al., is specifically addressed to the problem of recovering the gene products, providing a method for synthesis and secretion of the protein by a genetically modified bacterium.

The use of viruses in eukaryotic host-vector systems has been the subject of a considerable amount of recent investigation and speculation. However, viral vector systems also suffer from significant disadvantages and limitations which diminish their utility. For example, a number of eukaryotic viral vectors are either tumorgenic or oncogenic in mammalian systems, thereby creating a potential for serious health and safety problems associated with resultant gene products and accidental infection. Further, in some eukaryotic host-viral vector systems, the gene product itself exhibits antiviral activity, thereby decreasing the yield of that protein. Such was the case with the 80% reduction in the yield of simian virus 40 caused by only 100 units of interferon in the eukaryotic host-viral vector system described by D. Gheysen and W. Fiers, 1 J. Molec. Applied Genet. 385-394 (1982).

Another problem inherent in the use of eukaryotic host-viral vector systems is presented by the morphology of viruses. For example, because they have fewer restriction sites, it is easier to insert exogenous DNA into simple viruses at specific locations. However, eukaryotic genes are often too large to fit into simple viruses. Thus, because of the morphology of the virus, the amount of exogenous DNA which can be packaged into a simple virus is limited. On the other hand, it is more difficult to insert exogenous DNA into complex viruses at specific locations because they have many restriction sites.

The present invention overcomes many of the limitations discussed above by utilizing a baculovirus and a promoter within the baculovirus genome to produce a viral expression vector in a eukaryotic host-vector system. More particularly, it has been discovered that the baculovirus Autographa californica (AcMNPV) and its associated polyhedrin promoter may be utilized to produce a recombinant viral expression vector capable of extremely high levels of expression of a selected gene in a eukaryotic host insect cell. The resultant gene products of this system may be efficiently secreted into the cell medium, alleviating most difficulties associated with the recovery of protein products. Further, and more significantly, this system is not oncogenic or tumorgenic in mammals. The theoretical advantages of utilizing baculoviruses in a eukaryotic host-viral vector system are discussed in more detail by L. K. Miller, "A Virus Vector for Genetic Engineering in Invertebrates." In: Panopoulos, N.J. (Ed.), Genetic Engineering in the Plant Sciences (New York, Praeger Publishers, 1981), pp. 203-224.

In its broadest scope, the present invention provides methods for producing a viral transfer vector, a recombinant viral transfer vector, and a recombinant viral expression vector. The resultant recombinant viral expression vector is capable of expressing a selected gene in a host insect cell.

In accordance with the present invention, baculovirus DNA comprising a baculovirus gene or a portion thereof which includes a promoter of said baculovirus gene is cleaved to obtain a DNA fragment containing at least said promoter. In the preferred method, DNA comprising the polyhedrin gene and flanking DNA sequences of an appropriate baculovirus, such as the preferred baculovirus Autographa californica - (AcMNPV), is first isolated. The desired DNA is then cleaved by appropriate restriction procedures. This produces DNA fragments some of which comprise the polyhedrin promoter and at least one DNA sequence encoding for the polyhedrin protein or a portion thereof. One such DNA fragment is an EcoRI-I fragment comprising the polyhedrin promoter and DNA sequences coding for the polyhedrin protein.

A transfer vector is next prepared by inserting the DNA fragment described above into a suitable cloning vehicle, such as the plasmid pUC8. Accordingly, the preferred transfer vector, designated as a polyhedrin transfer vector, comprises a suitable cloning vehicle containing the polyhedrin promoter and an available site for cloning a selected gene or portion thereof such that the selected gene is under the transcriptional control of the polyhedrin promoter. The preferred transfer vector may or may not also contain DNA sequences coding for the polyhedrin protein or a portion thereof.

A recombinant transfer vector is thereafter prepared by inserting a selected gene or portion thereof into the available cloning site of the above-described transfer vector. Potentially any gene or genes may be cloned into the transfer vector of this invention and coupled with a baculovirus promoter sequence. Additionally, by appropriate recombinant DNA techniques, the DNA sequences encoding for polyhedrin protein may be deleted from the above-described preferred transfer vector such that the resultant cloned gene product will be the selected protein by itself. Alternatively, if no coding sequences for polyhedrin protein are deleted, or at least one coding sequence for polyhedrin protein is not deleted from the preferred transfer vector, the resultant cloned gene product will be a hybrid or fused protein comprising the selected protein and the polyhedrin protein or a portion thereof.

To produce the recombinant expression vector, the recombinant transfer vector is contacted with an appropriate baculovirus DNA so as to effect recombination, thereby incorporating the desired genetic material into the baculovirus genome. The preferred means of accomplishing recombination is by the well

EP 0 127 839 B1

known process of transfection. Since the process of transfection does not occur in 100 percent of the viruses, the result will be a mixture of nonrecombinants and recombinants.

Recombinant baculovirus expression vectors, capable of expressing the selected gene in host insect cells, are thereafter selected by appropriate screening or genetic selection techniques from this mixture of recombinant and nonrecombinant baculoviruses. One means of selecting the expression vector is made by identifying and isolating those viruses which lack viral occlusions in the nuclei of infected cells due to the insertional inactivation of the polyhedrin gene.

The present invention is also directed to the recombinant baculovirus expression vector produced by the method as described above. Such an expression vector comprises an infectious baculovirus containing at least one selected gene or portion thereof that is linked to the virus genome and is stable within it. During replication of the expression vector in insect cells or insects, the selected gene can be expressed either under the control of the baculovirus transcriptional signals or under the control of its own promoter. An exemplary baculovirus expression vector is the recombinant AcMNPV virus containing the gene for $\beta$-interferon, inserted into the AcMNPV genome in a location such that it is under the transcriptional control of the polyhedrin promoter. Potentially any baculovirus promoter and insect cell line can be used.

This invention is further directed to the transfer vector produce by the methods described above. The preferred polyhedrin transfer vector, comprising at least the polyhedrin promoter sequence and an available cloning site for insertion of a selected gene or portion thereof such that the selected gene is under the transcriptional control of the polyhedrin promoter, is used as an intermediate vehicle for the genetic manipulation of baculoviruses. The polyhedrin transfer vector may contain none, all, or a portion of the DNA sequences coding for the polyhedrin protein.

This invention is further directed to the recombinant transfer vector produced by the methods described above. The preferred recombinant transfer vector of this invention, comprising a selected gene or portion thereof coupled with the polyhedrin promoter, is used as a vehicle for incorporating desired genetic information into the baculovirus genome.

The present invention is predicated upon the use of a baculovirus promoter in a host-vector system to promote the expression of a selected gene in a eukaryotic host insect cell. In particular, because the polyhedrin protein is one of the most abundantly synthesized proteins within a viral-infected eukaryotic host cell, the preferred method involves the incorporation of a selected gene into an appropriate baculovirus genome such that the selected gene is coupled with the polyhedrin promoter. Such a recombinant baculovirus provides an effective mechanism for synthesis of a selected gene product. Accordingly, the present invention is of significant utility as extremely high levels of desired gene products, such as $\beta$-interferon, synthesized and efficiently secreted from host insect cells.

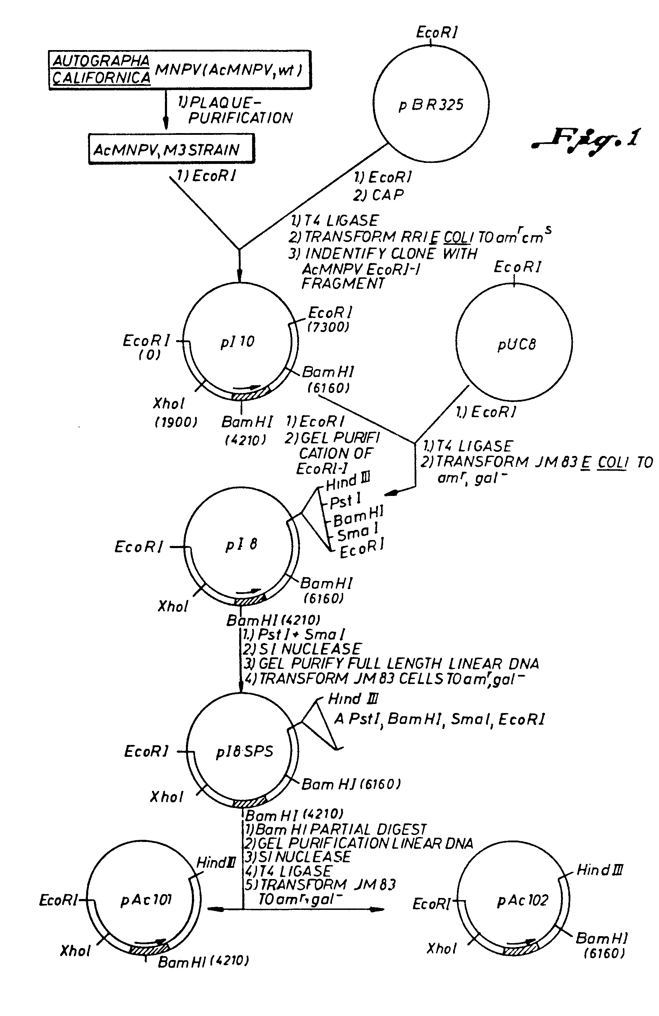

FIG. 1 depicts the scheme for the construction of a transfer vector, pAc101, starting with a plaque-purified strain of AcMNPV, M3, the plasmid pBR325, and the plasmid pUC8.

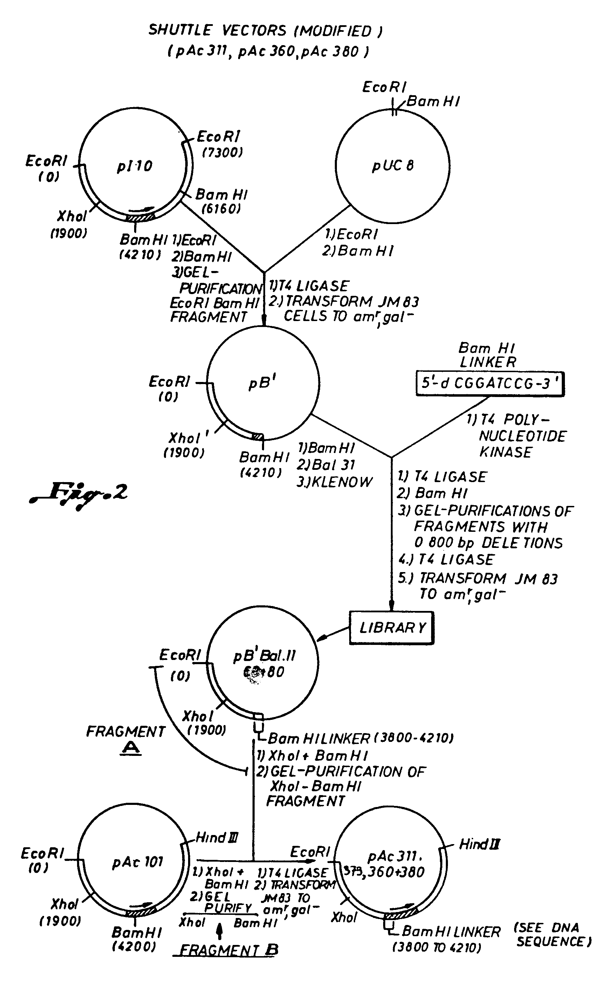

FIG. 2 depicts the scheme for constructing the modified transfer vectors pAc311, pAc360, pAc373 and pAc380 starting with the plasmids pI10, pUC8 and a synthetic BamHI linker, wherein the term "Library" represents a library of modified pB'Bal plasmids which may be constructed by inducing deletion mutations at each possible position in the polyhedrin gene. The plasmids pB'Bal 11, pB'Bal 60, pB'Bal 73 and pB'Bal 80 were then selected from this library of mutant plasmids for further modification into the transfer vectors pAc311, pAc360, pAc373 and pAc380.

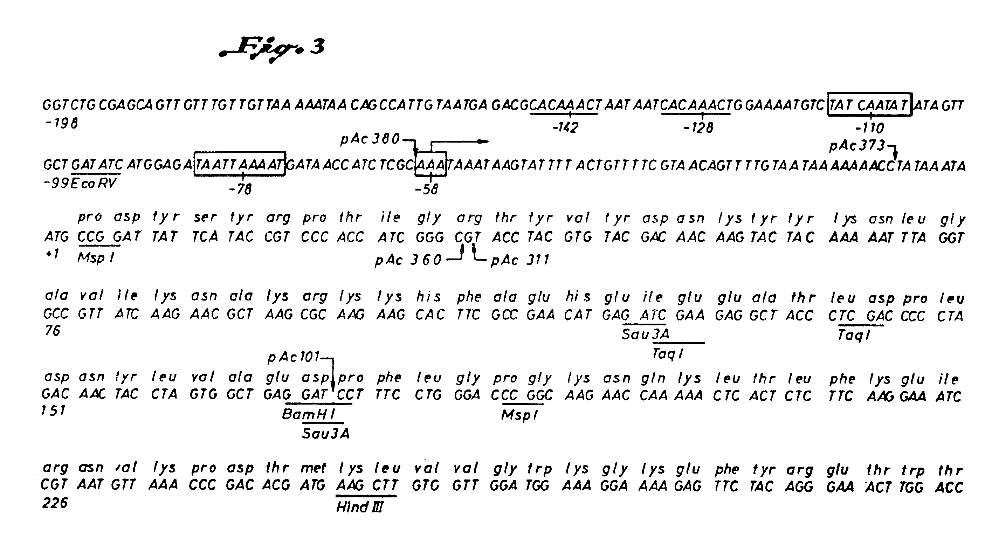

FIG. 3 schematically shows the partial nucleotide sequence of the polyhedrin gene of AcMNPV and the sequence immediately upstream of that gene. In addition to the location of the unique BamHI cloning site, the points at which the deletion mutations were induced to construct the transfer vectors pAc101, pAc311, pAc360, pAc373 and pAc380 are indicated by the arrows. The "TATA" and "CAAT" boxes of the polyhedrin gene are indicated by rectangles drawn around those sequences and the transcriptional start site of the gene is indicated by the asterisks. Fig. 3 also shows the EcoRV and the HindIII restriction sites located near the polyhedrin gene.

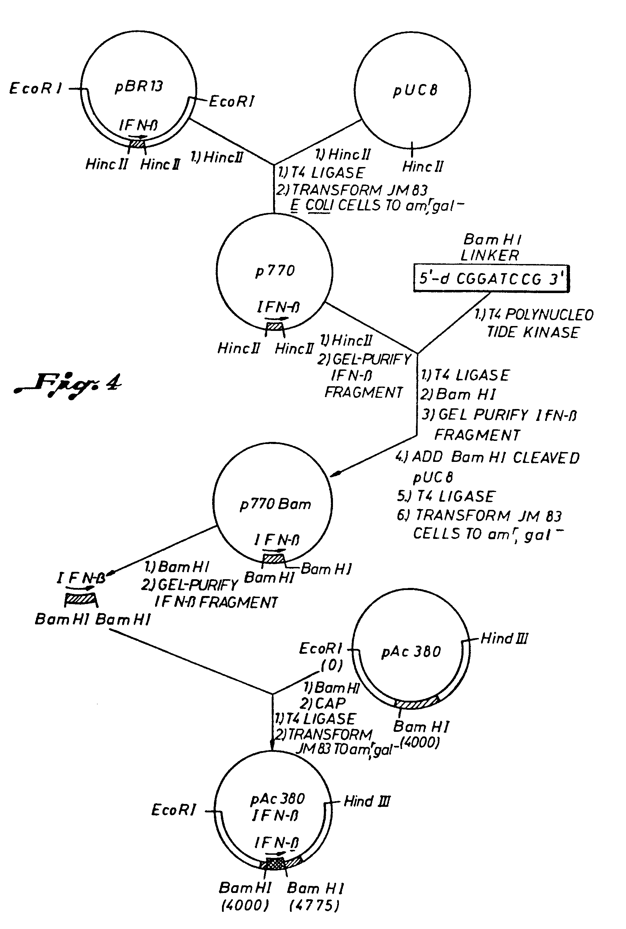

FIG. 4 depicts the scheme for the cloning of the preferred IFN-$\beta$ gene into the transfer vector pAc380 to construct the recombinant expression vector pAc380-IFN-$\beta$. Fig. 4 also shows the starting material plasmid pBR13, which contains the IFN-$\beta$ gene. The plasmid p770 contains the sequences for pUC8 and a 767 base pair HincII fragment flanked by synthetic octanucleotide BamHI linkers. This 767 base pair fragment contains the entire coding sequences for IFN-$\beta$.

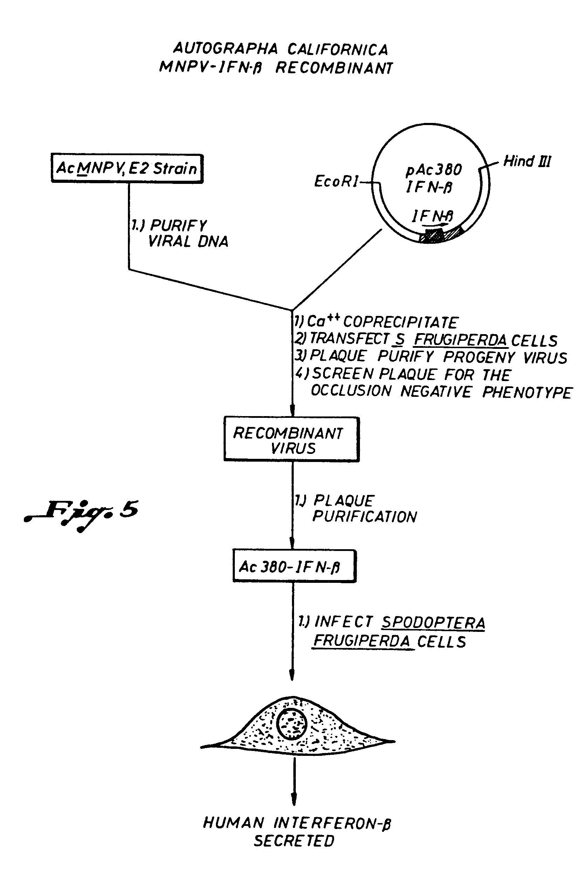

FIG. 5 depicts the scheme for the transfection of the recombinant expression vector pAc380-IFN-$\beta$ with baculoviruses in cultured Spodoptera frugiperda cells and the subsequent infection of cultured S. frugiperda cells with the plaque-purified recombinant baculoviruses.

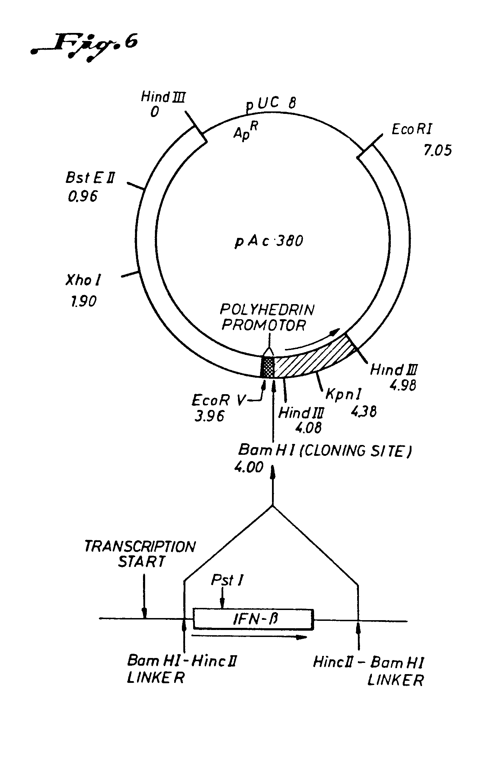

FIG. 6 shows the recombinant transfer vector pAc380-IFN-$\beta$ with the IFN-$\beta$ gene inserted at the BamHI cloning site of the EcoRI fragment of the AcMNPV genome. The polyhedrin promoter sequence is indicated

6

in solid black and the EcoRI-I sequence is shown as being incorporated into the plasmid pUC8.

The baculovirus used in the practice of the present invention is Autographa californica (AcMNPV). This baculovirus is characterized as follows.

In its naturally occurring, infectious form, AcMNPV is usually found in a viral occlusion. These viral occlusions usually contain several virus particles embedded in a paracrystalline protein matrix comprising a structured array of polyhedrin protein subunits. An occlusion is ingested by an appropriate host insect, and when it reaches the lumen of the gut, the alkaline conditions cause the disassociation of the occlusion to release the virus.

The viruses invade the cells of the gut wall, migrate to the nucleus of those cells and replicate. Two infectious forms are produced in these cells, the extra-cellular or nonoccluded virus form, and the occluded virus. The extracellular virus buds from the surface of the cell to infect other cells. Approximately twelve hours after infection, there is a decrease in extracellular virus budding, initiation of polyhedrin synthesis, and an increased production of occluded virus particles. Very large numbers of occlusions are produced in infected cells and tissues, ultimately lysing the insect. This occluded form of the virus is responsible for the spreading of infection to other insects.

The extracellular virus which is easily cultured in cell culture medium is used in the exemplary methods of the present inventon. The extracellular and occluded virus have the same genome, but exhibit different phenotypic properties.

The major structural component of these occlusons, polyhedrin, is a protein of approximately 29,000 molecular weight. Characterizaton of the AcMNPV genome indicates that the gene for AcMNPV polyhedrin maps to a contiguous DNA sequence of about 1200 base pairs in the EcoRI-I fragment at about 4000 base pairs down stream from the zero point of the DNA restriction map, see G. E. Smith, J. M. Vlak and M. D. Summers, 45 J. Virol. 215-225 (1983).

However, this reference does not disclose the nucleotide sequence of the polyhedrin gene so as to enable one of ordinary skill to insert a heterologous gene within the polyhedrin gene under the transcriptional control of the polyhedrin promoter and outside the gene sequence coding for the structural polyhedrin protein. This reference does not portray any available natural, or synthetic restriction site within the region from the transcriptional start to the ATG start of the polyhedrin structural gene. Nor does this reference provide guidance as to how to create a unique restriction site in that region.

A map of the entire AcMNPV genome may be found in J. M. Vlak and G. E. Smith, 4 J. Virol. 1118-1121 (1982) and the DNA sequence for the polyhedrin gene in Hooft van Iddekinge, G.E. Smith, and M.D. Summers. 131 Virology 561-565 (1983).

The structure and function of the polyhedrin protein are of considerable interest because it is one of the most highly expressed eukaryotic genes known. In Spodoptera frugiperda cells infected with AcMNPV, polyhedrin accumulates to such high levels that it forms 50% or more of the total mass of the protein in an infected cell and greater than 0.2 grams of polyhedrin protein per liter of infected cells is produced. The gene is also of interest for the following reasons: (1) the polyhedrin gene of AcMNPV contains DNA sequences that are highly conserved among baculoviruses, (2) recombination between closely related strains occurs at a high frequency in this region of the genome, thus allowing the segregation and expression of the polyhedrin gene in recombinant progeny, and (3) expression of the gene is host, tissue, and cell line dependent.

From the point of view of a genetic engineer, the polyhedrin gene is unnecessary because the virus is capable of replication in cell culture without it. Because of the high degree of expression of this gene and the fact that it is not essential for viral replication, the possibility of using the polyhedrin promoter and gene of AcMNPV as part of a system for the expression of a recombinant gene has been the source of considerable speculation (see E. B. Carstens, S. T. Tjia and W. Doerfler, 99 Virology 386-398 (1979); P. Dobos and M. A. Cochran, 103 Virology 446-464 (1980); H. A. Wood, 102 Virology 21-27 (1980); J. E. Maruniak and M. D. Summers, 109 Virology 25-34 (1981); L. K. Miller, "A Virus Vector for Genetic Engineering in Invertebrates," In: N.J. Panopoulos (Ed.), Genetic Engineering in the Plant Sciences (New York, Praeger Publishers, 1981), pp. 203-224; and L. K. Miller, et al., 219 Science 715-721 (1983); G. E. Smith, M. J. Fraser, and M. D. Summers, J. Virol. 584-593 (1983)). However, prior to the present invention, no one has been able to develop such a system.

Experimentation by applicants indicates that another gene product, 10K, is also expressed at a high level comparable to polyhedrin (G. E. Smith, J. M. Vlak and M. D. Summers, 45 J. Virol. 215-225 (1983)). The 10K protein produced is apparently nonstructural, is produced late in infection and in large quantity. The location of the 10K AcMNPV protein gene maps to the HindIII fragments P and Q. Like the polyhedrin promoter and structural gene, this promoter has been the object of speculation as to its advantageous use as part of a system using AcMNPV for the expression of a recombinant gene. However, until the present invention, a system which would allow such advantageous use of this promoter had not been developed.

According to the illustrative method of this invention, the particular strains of AcMNPV, M3 or E2, are utilized. However, those skilled in the art who have the benefit of this disclosure will recognize that other baculoviruses and other baculovirus strains are suitable to produce recombinant baculovirus transfer and expression vectors. In particular, the closely related and naturally occurring baculovirus strains Trichoplusia ni MNPV, Rachiplusia ou MNPV, Galleria mellonella MNPV and any plague-purified strains such as the E2, R9, S1 and S3 strains of AcMNPV characterized in this laboratory and described in G. E. Smith and M. D. Summers, 30 J. Virol. 828-838 (1979) and G. E. Smith and M. D. Summers, 33 J. Virol. 311-319 (1980) be utilized to advantage. Further descriptions of these and other strains are found in G. E. Smith and M. D. Summers, 89 Virology 517-527 (1978).

In accordance with the methods of this invention, it is the polyhedrin structural gene and promoter which are utilized to advantage. This gene has been mapped by S1 nuclease experiments. The nucleotide sequence of the 5' end of the polyhedrin coding region and 200 bases upstream from the start of transcription are shown in Fig. 3. The site indicated in Fig. 3 is the most frequently used transcriptional start site for polyhedrin mRNA.

An ATG translation start signal (with the A residue assigned position +1) occurs approximately 58 bases from the transcriptional start site, followed by in open leading up to and including the HindIII site at 255. The "TATA" and "CAAT" boxes found in similar positions in many eukaryotic structural genes are located between 25 and 35 bases and between 60 and 70 bases upstream from the transcriptional start site respectively. Centered at 78 and 90 bases upstream from the transcriptional start site are the direct repeated sequences "CACAAACT". In addition, the AcMNPV polyhedrin gene has a 58 base nontranslated leader sequence preceding the translational start codon and, as suggested by appropriate experimental procedures on AcMNPV polyhedrin mRNA, there are no intervening sequences. See, Smith, Vlak and Summers, supra and G. F. Rohrman, et al., 121 Virology 51-60 (1982).

In the practice of the present invention, DNA having a polyhedrin gene is isolated and purifed from the baculovirus AcMNPV. It will be recognized by those skilled in the art who have the benefit of this disclosure that this gene could be isolated from any baculovirus which possesses a polyhedrin gene, particularly the related strains described above.

The desired DNA is then digested with EcoRI restriction endonuclease by appropriate restriction procedures to produce a 7.3 kilobase EcoRI-I fragment or other appropriate fragments comprising the polyhedrin gene.

The EcoRI-I fragment described above is thereafter cloned into the EcoRI site of an appropriate cloning vehicle to produce a transfer vector. Because the AcMNPV genome has no known unique restriction sites into which selected genes may be effectively introduced in a site-specific manner, it is necessary to construct chimeric plasmid vectors (transfer vectors) to serve as intermediate vehicles for gene transfer. Therefore, to incorporate selected genes into the viral genome adjacent to the polyhedrin promoter sequence, a transfer vector is constructed, designated as the polyhedrin transfer vector, which comprises the polyhedrin gene, a cloning site located such that a gene properly inserted into the site will be under the control of the polyhedrin promoter, and flanking viral DNA linked to either side of the polyhedrin gene. The construction of this transfer vector is schematically shown in Figs. 1 and 2. Additionally, it should be noted that the presence of flanking viral DNA facilitates recombination with the wild type baculovirus, allowing the transfer of a selected gene into a replicating viral genome.

Accordingly, the EcoRI-I fragment described above is cloned and subcloned into the plasmids pBR325 and pUC8, respectively. Two BamHI restriction sites in the EcoRI-I fragment may thereafter be removed so as to produce a polyhedrin transfer vector, designated as pAc101, having a single BamHI cloning site located in the 3' direction downstream from the polyhedrin promoter sequence approximately 220 bases from the translational start site of the polyhedrin gene (See Fig. 3). While plasmids pBR325 and pUC6 are the plasmids utilized for construction of the polyhedrin transfer vector, it will be recognized by those skilled in the art that other suitable cloning vehicles can be utilized provided the polyhedrin gene and flanking viral DNA be functionally incorporated.

The polyhedrin transfer vector may thereafter be modified for insertion of a selected gene by deleting some or all of the sequences encoding for polyhedrin synthesis near the transcriptional start site or from about -58 to the end of the polyhedrin gene (See Fig. 3). A DNA linker, comprising a natural or synthetic oligonucleotide bearing the BamHI restriction site sequence, is then inserted at the site of the deletion to allow the coupling of DNA segments at that restriction site. The modification of the transfer vectors is shown schematically in Fig. 2 and the means of deleting polyhedrin coding sequences is by in vitro mutagenesis. However, it will be recognized by those skilled in the art who have the benefit of this disclosure that alternative procedures are available to delete part or all of the polyhedrin coding sequences, that alternative synthetic or natural oligonucleotide linker sequences could be inserted at the site of the deletion, and that alternative modified polyhedrin transfer vectors into which a selected gene or portion thereof may be incorporated may be suitably utilized in the present invention.

In accordance with standard cloning techniques, a selected gene, such as IFN-$\beta$ gene encoding for human $\beta$-interferon synthesis, CAT gene encoding for chloramphenicol acetyltransferase synthesis, and IL2 gene encoding for human interleukin-2 synthesis, is thereafter inserted into the polyhedrin transfer vector at the available restriction site to produce a recombinant transfer vector. Insertion of the $\beta$-interferon gene into the transfer vector pAc380 is shown schematically in Fig. 4.

Further, while the IFN-$\beta$, CAT, and interleukin-2 are exemplary genes for cloning into the polyhedrin transfer vector and coupling with the polyhedrin promoter sequence, it will be recognized that potentially any selected gene may be utilized in the present invention or in its above described alternative forms.

The hybrid polyhedrin-selected gene, gene of the recombinant transfer vector is thereafter transferred into the genome of an appropriate baculovirus, such as the baculovirus AcMNPV to produce a recombinant viral expression vector capable of expressing the gene encoding for $\beta$-interferon in a host insect cell. Transfer of the hybrid gene is accomplished by the process of transfection in host insect cells, such as Spodoptera frugiperda. J. P. Burand, et al., 101 Virology 286-290 (1980). This process is shown schematically in Fig. 5, utilizing the recombinant transfer vector pAc380-IFN-$\beta$. During replication of the AcMNPV DNA after transfection, the hybrid gene is transferred to AcMNPV DNA by recombination between the recombinant transfer vector and AcMNPV DNA. Accordingly, a mixture is produced comprising nonrecombinant and recombinant baculoviruses of which the latter is capable of expressing the IFN-$\beta$ gene. While transfection is the preferred process for transfer of the hybrid gene into the baculovirus genome, it will be understood by those skilled in the art that other procedures are suitable to insert the hybrid gene into the baculovirus genome. Further, recombination can be accomplished between the recombinant transfer vector and other strains of baculoviruses, as long as there is sufficient homology between the sequence of the hybrid gene and the corresponding sequence of the other strain to allow recombination to occur. For instance, such recombination should occur between genes isolated from any of the above-described strains Trichoplusia ni MNPV, Rachiplusia ou MNPV, and Galleria mellonella MNPV, as well as the AcMNPV strains E2, R9, S1 and S3. It is also possible for recombination to occur in regions of the genome which apparently do not contain homologous sequences. The mechanism for this is not understood.

The recombinant AcMNPV expression vector, comprising the hybrid polyhedrin-selected gene, gene incorporated into the AcMNPV genome, is thereafter selected from the mixture of nonrecombinant and recombinant baculoviruses. One of the means of selection is by screening for plaques formed by host insect cells infected with viruses that do not produce viral occlusions (designated as 0⁻). Selection is facilitated in this manner because recombinant viruses arc defective in the production of viral occlusions due to the insertional inactivation of the polyhedrin gene. Of the viral plaques produced from the progeny virus from transfected cells, an average of 0.5% will be from putative recombinant 0⁻ viruses. Accordingly, the DNA from an 0⁻ plaque-forming recombinant virus is thereafter purified and analyzed with appropriate restriction enzymes to confirm that the recombinant AcMNPV vector has an insertion of the selected gene in the proper EcoRI-I location.

The above-described selection procedure provides an effective and convenient means for selection of recombinant baculovirus-selected gene expression vectors, however it will be recognized by those skilled in the art that alternative selection procedures may also be utilized in accordance with the present invention. A relatively convenient procedure for detection in situ of foreign DNA in eukaryotic cells (i.e., hybridizing a labeled DNA probe to viral DNA present in plaques produced in infected animal cells) is described by Villarreal and Berg, 196 Science 183-186 (1977) and Hayday, et al., 15 Gene 53-65 (1981).

Expression of the selected gene is accomplished by infecting susceptible host insect cells, such as the Spodoptera frugiperda, with the recombinant baculovirus-selected gene expression vector in an appropriate medium for growth. An AcMNPV expression vector is propagated in insect cells or insects through replication and assembly of infectious virus particles. These infectious AcMNPV expression vectors can be used to produce the selected gene in suitable insect cells, thus facilitating the efficient expression of the selected DNA sequence in the infected cell.

During infection, AcMNPV expression vector-specific mRNA will be produced that is complementary to the DNA sequence of the selected gene. The vector-specific mRNA will usually be translated in infected cells to produce a protein that is completely or partially coded for by the selected gene and in some instances, the selected gene product will undergo processing such as glycosylation, secretion, phosporylation, or other post-translational modifications.

Whether the gene product produced by the recombinant AcMNPV expression vector consists of the amino acid sequences of only the selected protein, is a hybrid protein containing one or more additional amino acid residues derived from the amino terminal end of AcMNPV polyhedrin, or whether both selected and hybrid protein products are produced is dependent upon the manner in which the polyhedrin transfer vectors are modified. If only a single translational start signal (ATG) derived from the selected gene is present in the hybrid gene sequences, and the selected gene is present in the hybrid gene sequences between about the -75 and +1 positions, then only the selected protein, such as $\beta$-interferon, will be produced (see Fig. 3). Alternatively, if the selected gene is fused to the polyhedrin promotor such that there are two translational start sites, the polyhedrin ATG signal at +1 and the selected gene ATG signal at between +3 and the end of the polyhedrin coding sequences, both the hybrid and selected proteins may be produced. However, the proportion of each protein produced may vary depending on the position of the second ATG signal and the nature of the selected gene sequences. Alternatively, if a gene is fused to the polyhedrin promotor without its own ATG start signal, then it will require that either a synthetic ATG or the polyhedrin ATG translation start signal be fused to the protein coding sequences in such a way as to maintain the correct translational reading frame for the selected gene. The protein products that will be produced will, again, depend upon the factors described above.

## DEPOSIT OF TRANSFER VECTORS AND RECOMBINANT EXPRESSION VECTOR

The recombinant baculovirus expression vector Ac380-IFN-$\beta$ was deposited with the American Type Culture Collection (Rockville, Maryland) on May 13, 1983, and assigned accession number ATCC 40071. The modified polyhedrin transfer vectors pAc380 plasmid in E. coli K-12 and the recombinant transfer vector pAc380-IFN-$\beta$ in E. coli K-12 were both deposited with the Agricultural Research Culture Collection (Peoria, Illinois) on May 13, 1983, and assigned the accession numbers NRRL B-15428 and NRRL B-15427, respectively. The modified polyhedrin transfer vector pAc373 was deposited with the Agricultural Research Culture Collection on May 7, 1984, and assigned accession number NRRL B-15778.

## STARTING MATERIALS AND METHODS

### Plasmid DNA

The plasmids used in the following examples were pBR325 and pUC8 in E. coli, and were obtained from Bethesda Research Labs, Gaithersburg, Maryland.

### Viral DNA

AcMNPV, strain M3, used in the examples as the original source of viral DNA, as well as AcMNPV, strain E2 and wild type, were isolated in this laboratory according to the techniques described in G. E. Smith and M.D. Summers, 89 Virology 517-520 (1978) and G. E. Smith and M.D. Summers, 39 J. Virol 125-137 (1981).

### IFN-$\beta$ DNA

The DNA fragment comprising the IFN-$\beta$ gene used in the examples was isolated from the plasmid pBR13, obtained from Dr. John Collins, Gesellschaft fur Biotechnologische Forschung (GBF), Braunschweig Stockhein, West Germany.

### CAT DNA

The DNA fragment containing the chloramphenicol acetyltransferase (CAT) gene was isolated from plasmid pBR328 obtained from Drs. Bernard Moss and Mark Cochran, National Cancer Institue, Bethesda, Maryland.

### IL2 DNA

The DNA fragment containing the gene coding for human interleukin-2 (IL2) was isolated from plasmid pIL2-2B obtained from Drs. Grace Ju and Peter Lomedico, Hoffmann LaRoche Research Center, Nutley, New Jersey.

### Bacterial Cells

E. coli JM83, used in the examples for cloning pUC8 plasmids, was obtained from Bethesda Research Labs, Gaithersburg, Maryland.

E. coli RR1, used in the examples for cloning pBR325 plasmids, was obtained from Dr. Savio Woo, Baylor College of Medicine, Houston, Texas.

Enzymes

The following restriction endonucleases were obtained from Bethesda Research Laboratories, Gaithersburg, Maryland, and used according to that supplier's recommendations: EcoRI, XhoI, BamHI, SmaI, PstI, BstEII, EcoRV, KpnI and HindIII. The following enzymes were also obtained from Bethesda Research Laboratories: calf intestinal alkaline phosphatase (CAP), DNA ligase, Ba131 exonuclease, S1 nuclease and T4 polynucleotide kinase.

Methods

The standard methods used in accordance with the cloning procedures set forth in the examples are described in T. Maniatis, E. F. Fritsch and J. Sambrook, Molecular Cloning: A Laboratory Manual, Cold Spring Harbor Laboratory, 1982. This reference includes procedures for the following standard methods: cloning procedures with E. coli plasmids, transformation of E. coli cells, plasmid DNA purificaton, phenol extraction of DNA, ethanol precipitation of DNA, agarose gel electrophoresis, purification of DNA fragments from agarose gels, and restriction endonuclease and other DNA-modifying enzyme reactions. In all cases, DNA was purified by phenol extraction followed by ethanol precipitation.

Virus stocks used in the examples were prepared and titrated in Spodoptera frugiperda cells (IPLB-Sf 21-AE) with TNM-FH medium (see W. F. Hink, 226 Nature (London) 466-467 (1970)) plus 10% fetal bovine serum. The procedures for the cultivation of viruses and cells are described in L. E. Volkman and M. D. Summers, 19 J. Virol. 820-832 (1975) and L. E. Volkman, M. D. Summers and C. H. Hsieh, 19 J. Virol. 820-832 (1976). Viral growth kinetics were determined as described by Volkman, et al., supra, using S. frugiperda and a 1.5% agarose overlay.

EXAMPLE I

CONSTRUCTION OF POLTHEDRIN TRANSFER VECTOR

To construct a polyhedrin transfer vector according to the present invention, a DNA fragment comprising the AcMNPV polyhedrin gene was cloned into the EcoRI site of the plasmid pUC8. This was accomplished by using a plaque-purified strain of AcMNPV, designated M3 (G. E. Smith and M. D. Summers, 89 Virology 517-527 (1978)), as the original source of the viral DNA. AcMNPV DNA was extracted from the virus and purified by equilibrium centrifugation in cesium chloride density gradients as described in the above-referenced reference by Smith and Summers. AcMNPV DNA was digested to completion with EcoRI restriction endonuclease. The resulting AcMNPV EcoRI-I fragment was first cloned into pBR325 to form pl10, then subcloned into pUC8 using standard cloning procedures to form pl8 (see Fig. 1).

The recombinant plasmid pl8 has three BamHI recognition sites (see Fig. 1): one in the polyhedrin gene at position 4210, one in pUC8 at 7300 and one in the EcoRI-I fragment at positon 6160. The sites at 6160 and 7300 are removed from pl8 so that the desired gene, such as the exemplary CAT, IL2 or IFN-$\beta$ genes, may conveniently be cloned into pl8 in the desired location (position 4210) adjacent to the polyhedrin promoter.

The pUC8 BamHI restriction site in pl8 at about 7300, which is not located in the polyhedrin gene, was removed as follows: 10 ug of pl8 was digested with PstI and SmaI, the DNA purified, and resuspended in S1 nuclease buffer (0.03 M sodium acetate, pH 4.4, 0.25 M NaCl and 0.0045 M ZnCl$_2$) plus 500 units S1 nuclease/ml. The reaction mix was incubated at 37°C for 15 minutes, then the DNA electrophoresed in an 0.8% agarose gel. High molecular weight linear DNA was purified from the gel and used to transform E. coli JM83 cells to ampicillin resistant (am$^r$), galactosidase negative (gal$^-$). A plasmid was isolated that was missing the PstI, BamHI, SmaI, and EcoRI sites in the pUC8 cloning region. This plasmid was designated pl8SPS (see Fig. 1).

The BamHI site at location 6160 (Smith, G.E., J.M. Vlak and M.D. Summers, J. Virol. 45: 215-225) in AcMNPV EcoRI-I (a part of pl8SPS) was removed as follows: 10 ug of pl8SPS was mixed with 10 units of BamHI in 50 ul of buffer and incubated at 37°C. 10 ul aliquots were removed after 3, 6, 12, 20, and 30 minutes and the reactions stopped by adding EDTA to 10 mM. The aliquots were pooled and electrophoresed in 0.7% agarose. Full length linear DNA was isolated from the gels, treated with S1 nuclease as above, then circularized with T4 DNA ligase. JM83 cells were transformed to am$^r$, gal$^-$ and a recombinant plasmid was identified that was missing the BamHI restriction site at position 6160. This plasmid has a single BamHI cloning site at position 4210 located +175 bases from the translation start site of the polyhedrin gene (see Fig. 3) and was designated pAc101, the "parent" AcMNPV polyhedrin gene transfer vector (Fig. 1).

EXAMPLE II

MODIFICATION OF TRANSFER VECTOR

In order to determine suitable locations in the polyhedrin gene for the insertion of a selected gene, a number of transfer vectors were constructed in addition to pAc101 (see Fig. 2). These transfer vectors were constructed by deleting some or all of the DNA sequence that encodes the 86 amino-terminal residues of polyhedrin and the 5' non-translated polyhedrin mRNA sequences, and then inserting an oligonucleotide synthetic DNA linker with a BamHI recognition site at the site of the deletion by the following procedures.

In the same manner as set forth in Example I, the EcoRI to BamHI fragment (0 to 4210) from pl10 was sub-cloned into pUC8 and the resulting plasmid was designed pB' (see Fig. 2). This fragment contains polyhedrin gene up to +175 and about 4000 base pairs of AcMNPV DNA sequences in addition to the polyhedrin gene. Deletions around the BamHI site at position 4210 were then introduced into pB' as follows (Fig. 2): 40 ug of pB' was digested with BamHI, the DNA was purified, and electrophoresed on an 0.7% agarose gel. The linear DNA fragment was extracted from the gel and incubated in 100 ul of buffer with 0.5 units of Ba131 exonuclease for 40 minutes at 300 C. 10 ul aliquots were collected at 1, 2, 5, 10, 15, 20, 25, 30, 35 and 40 minute intervals and the reaction stopped by adding 10 ul 0.25 M EDTA to each aliquot. The aliquots were pooled and the DNA purified. The ends of the DNA were repaired by incubating in 100 ul of buffer for 30 minutes at 23°C with 4 units E. coli DNA polymerase (Klenow fragment). The DNA was purified and 1 ug of phosphorylated BamHI linker (5'-pCGGATCCG-3') was added plus 20 units T4 DNA ligase in 100 ul reaction mix. After incubation for 2 hours at room temperature, the DNA was purified.

Next, the DNA pellet was resuspended in 100 ul of buffer plus 20 units of BamHI and digested for 4 hours at 37° C. The digested DNA was electrophoresed in 0.7% agarose and pB' truncated plasmids with up to 800 base pair deletions were purified from the gel. The DNA was circularized with T4 DNA ligase and used to transform JM83 cells to am$^r$, gal$^-$. The resulting clones constituted the "LIBRARY" of mutant plasmids designated pB'Bal 1, 2, 3, and so on depending upon the location of the BamHI recognition site.

Several pB'Bal deletion mutant plasmids were selected and the XhoI (1900) to the BamHI linker (at positions 4000 to 4210) fragment from each was purified from an agarose gel (A fragments)(Fig. 2). The XhoI (1900) to BamHI (4210) fragment was removed from pAc101 and the remaining sequences purified from an agarose gel (B fragment)(Fig. 2). About 0.1 ug of each of the A fragments were mixed with 0.1 ug of Fragment B, joined by incubating in 20 ul of buffer with 1 unit of T4 DNA ligase, then used to transform JM83 cells to am$^r$, gal$^-$. The resulting plasmids were the modified transfer vectors and referred to as, for example, pAc311 if derived from pB'Bal 11, pAc360 if derived from pB'Bal 60, and so on. In this manner, a group of "modified" transfer vectors was constructed with representatives having BamHI cloning sites at positions between +175 and -100 in the polyhedrin gene. The location of the BamHI recognition site in four of the modified transfer vectors, pAc380, pAc373, pAc311 and pAc360, as well as its location in the parent transfer vector, pAc101, is shown in Fig. 3.

EXAMPLE III

CONSTRUCTING RECOMBINANT TRANSFER VECTOR COMPRISING POLYHEDRIN - IFN-$\beta$ GENE

Any one of the transfer vectors prepared according to the methods of Examples I or II may be utilized in the construction of a recombinant transfer vector in which the desired gene is linked to the polyhedrin gene at various locations, depending upon the particular modified transfer vector utilized. The insertion of the IFN-$\beta$ gene encoding for human $\beta$-interferon synthesis into one of the modified transfer vectors, pAc380, at the single BamHI cloning site using standard cloning techniques as referenced above to form the plasmid designated pAc380-IFN-$\beta$ is shown schematically in Fig. 4.

The IFN-$\beta$ gene may be characterized as a 0.767 kilobase HincII fragment, obtained from a genomic clone of human IFN-$\beta$ (designated pBR13, see H. Hauser, et al., 297 Nature (London) 650-654 (1982) and G. Gross, et al., 9 Nucleic Acids Res. 2495-2507 (1981)), and containing the entire protein coding sequences for IFN-β, three additional bases before the ATG translation start signal, and all of the non-translated 3' sequences including the signal for polyadenylation. The nucleotide sequence for IFN-β and the location of various transcription and translation signals are described by R. Derynck, et al., 285 Nature (London) 542-547 (1980); G. Gross, et al., 9 Nucl. Acids. Res. 2495-2507 (1981); and S. Ohno and T. Taniguchi, 78 Proc. Natl. Acad. Sci. USA 3505-3509 (1981).

The HincII DNA fragment comprising the IFN-β gene is inserted into the available BamHI restriction site of pAc380 using synthetic oligonucleotide linkers on the IFN-β gene fragment such that insertion is adjacent to the AcMNPV polyhedrin promoter and in the same 5' to 3' orientation as the polyhedrin gene.

In essentially the same manner, the IFN-β gene was cloned into the other modified transfer vectors pAc311, pAc360 and pAc373 and the parent transfer vector pAc101 to form the recombinant transfer vectors designated pAc311-IFN-β, pAc360-IFN-β, pAc373-IFN-β and pAc101-IFN-β, respectively. Potentially, that gene, or any other selected gene, could be cloned into any of the transfer vectors with the BamHI recognition site or any other suitable restriction endonuclease cleavage site inserted at any location in the transfer vector. Further, it is not necessary to delete part or all the polyhedrin structural sequence to insert the BamHI recognition site, as suitable restriction endonuclease cloning sites could be induced at any point in any fragment of the AcMNPV genome which could be incorporated into a plasmid as outlined in Example I.

## EXAMPLE IV

### TRANSFER OF POLYHEDRIN - IFN-β GENE TO AcMNPV GENOME

Any of the recombinant transfer vectors prepared by the method of Example III can thereafter be used to transfer the IFN-β gene into the genome of AcMNPV to form a recombinant baculovirus expression vector. Transfer was effected by transfection in the presence of $Ca^{++}$ and susceptible insect host cells such as S. frugiperda.

A modification of the method described by F. L. Graham and A. J. Van Der Eb, 52 Virology 456-467 (1973) was utilized as follows: DNA extracted and purified from AcMNPV (1 ug) was mixed with 1 to 10 ug of the recombinant transfer vector DNA, in particular, the recombinant transfer vector PAc380-IFN-β and brought to 950 ul in 1-HEPES (N-2-hydroxyethylpiperazine-N'-2-ethanesulfonic acid)-buffered saline (pH 7.05) with 15 ug of carrier calf thymus DNA per milliliter. While the mixture was being stirred in a Vortex mixer, 50 ml of 2.5 M $CaCl_2$ was added dropwise, and a precipitate was allowed to form at room temperature for 30 minutes. One milliliter of precipitated DNA was added to susceptible insect host cells, in this case, S. frugiperda, in 2 ml of medium in 60 mm culture plates. After 4 hours, the cell monolayer was washed with medium and incubated in 2 ml of medium with 10% fetal bovine serum for 3 days. The resulting progeny constituted a mixture of recombinant and nonrecombinant AcMNPV viruses.

### EXAMPLE V

### SELECTION OF RECOMBINANT AcMNPV EXPRESSION VECTOR

It was next necessary to isolate an AcMNPV recombinant expression vector from the resultant mixture of non-recombinant and recombinant baculoviruses described by Example IV. The method by which this isolation was accomplished in those recombinants in which all or a portion of the polyhedrin structural gene was deleted takes advantage of the facts (1) that no polyhedrin will be produced by these viruses and (2) the non-occluded (lacking a polyhedrin coat) viral form is viable and infectious in insect cell culture.

In those recombinant AcMNPV viruses in which all or a portion of the polyhedrin structural gene was deleted, e.g., those resulting from transfection with the recombinant transfer vectors pAc101-IFN-β, pAc311-IFN-β, pAc360-IFN-β, pAc373-IFN-β and pAc380-IFN-β, the recombinant AcMNPV viruses are isolated as follows.

The extracellular virus present in the medium at 3 days post transfection was used in a standard AcMNPV polyhedrin plaque assay described by L. E. Volkman, M. D. Summers and C. H. Hsieh, 19 J. Virol. 820-832 (1976). The plaques that developed were either from cells infected with nonrecombinant AcMNPV, which produced viral occlusions, or the result of cells infected with recombinant AcMNPV virus, which did not produce viral occlusions. The latter type plaque ($0^-$ plaques) was initially distinguished from the former ($0^+$ plaques) by their appearance under a low power binocular microscope. $0^-$ plaques were marked and examined with the aid of an inverted phase microscope, in which individual viral occlusions, if

EP 0 127 839 B1

present, could be seen in the nucleus of the infected cells.

The virus from an 0⁻ plaque was plaque purified and the DNA analyzed with the appropriate restriction enzymes to confirm that the recombinant AcMNPV vector had an insertion of the foreign gene in the proper location in EcoRI-I. No other changes in the viral genomes were, detected. Large stocks of the desired recombinant virus were then obtained by infecting susceptible insect cells using standard procedures.

EXAMPLE VI

PRODUCTION OF β-INTERFERON USING AcMNPV RECOMBINANT EXPRESSION VECTOR

In order to produce the desired gene product, the recombinant baculovirus must be infected in susceptible host insect cells. The following procedure was utilized for the exemplary AcMNPV expression vectors formed by recombination with the recombinant transfer vectors pAc101-IFN-β, pAc311-IFN-β, pAc360-IFN-β, pAc373-IFN-β and pAc380-IFN-β (see Fig. 5).

Susceptible S. frugiperda host cells were first grown either in suspension culture or in monolayers to a density of from 1 to 5 x $10^6$ cells/ml (the concentration of cells for optimal expression may vary for each different AcMNPV expression vector). The growth medium was removed and replaced with medium containing from 0.1 to 100 (optimal concentration may vary) plaque forming units of recombinant virus per cell, e.g., Ac380-IFN-β (Fig. 5) for about 1 hour at 23°C. The inoculum may be left on the cells or removed and replaced with the appropriate amount of fresh medium.

The protein from the cloned IFN-β gene was produced in S. frugiperda cells infected with the Ac-380-IFN-β expression vector from about 12 hours post infection to 3 days post infection. The entire infection process, including viral protein synthesis, viral assembly, and cell lysis was complete at about 72 hours. There was a marked reduction in protein synthesis between 50 and 60 hours post infection, and cell lysis was first detected at about 50 hours post infection. The kinetics of the expression of the IFN-β gene varied depending upon the host cell and the site at which the gene was cloned into the polyhedrin gene.

The kinetics of IFN-β expression for each of the recombinant viruses is summarized in Table 1. Significant levels of interferon were detected by 48 hours post infection in cells infected with each of the expression vectors. Of the expression vectors examined, Ac373-IFN-β and Ac380-IFN-β produced the highest titer of interferon activity (Table 1).

Intracellular levels of interferon activity were also measured and are reported in Table 1. Less than 5% of the total interferon activity remained inside Ac373-IFN-β and Ac380-IFN-β infected cells at 48 hours post infection, demonstrating that IFN-β secretory signals were recognized and the protein was efficiently released into the media during infection. At 12 hours post infection, the medium from Ac373-IFN-β and Ac380-IFN-β infected cells had about 10,000 IU/ml of interferon and increased to a maximum of nearly 5 x $10^6$ IU/ml by 42 hours post infection.

Table 1

| The kinetics of interferon expression were examined in S. frugiperda cells infected with the various expresssion vectors. The results of these experiments were as follows: | | | | |
|---|---|---|---|---|
| | Inside Cell | Outside cell | | |
| Viruses | $10^6$ IU/$10^7$ cells | $10^6$ IU/$10^7$ cells | $10^6$ IU/liter | (%) |
| Ac380-IFN-β | 0.98 | 50.7 | 5,070 | 98.1 |
| Ac373-IFN-β | 0.98 | 50.7 | 5,070 | 98.1 |
| Ac360-IFN-β | 0.96 | 20.8 | 2,080 | 95.6 |
| AC311-IFN-β | 0.013 | 1.4 | 140 | 99.1 |
| AC101-IFN-β | 0.043 | 0.007 | 0.7 | 14.0 |
| AcMNPV | 0 | 0 | 0 | 0 |

The synthesis of polyhedrin in AcMNPV infected cells is known to follow a similar temporal pattern of expression. Although less interferon was produced in Ac360-IFN-β and even less in Ac311-IFN-β virus infected cells, greater than 95% of the activity was present in the medium (Table 1). A relatively low level of interferon was detected in Ac101-IFN-β infected cells, most of which was intracellular (Table 1).

The titer of recombinant virus infected cells reached a maximum of 3 to 8 x $10^8$ plaque forming units per milliliter of medium, which is typical of AcMNPV infected cells. Thus, it appears that the insertion of the

14

IFN-$\beta$ gene into the polyhedrin gene had no major effect on the replication of the virus. To test this, 2 x $10^6$ S. frugiperda cells were treated for 12 hours with up to 5 x $10^6$ IU/ml of interferon produced in Ac380-IFN-infected cell medium or 5 x $10^3$ IU/ml of an international standard of human interferon, then the treated cells were infected with 100 plaque forming units of AcMNPV or Ac380-IFN-$\beta$. Exposure of the cells to interferon had no measurable effect on the number of virus plaques that developed.

The virus plaque-reduction assay in human amnionic WISH cells challenged with vesicular stomatitis virus was used to assay for interferon activity. If virus particles were removed by centrifugation, no interferon activity was measured in medium from AcMNPV infected cells. However, if AcMNPV virus-containing media was used during interferon assays, 1000 to 3000 international reference units (IU)/ml of interferon were produced, indicating that AcMNPV virions apparently induced endogeonous interferon in WISH cells. Because many species of enveloped viruses are known to induce interferon production in human cells, these results were expected. To avoid this effect, all subsequent samples were centrifuged before testing.

An experiment was conducted to determine whether the serum albumin (6 mg/ml) and calf serum (10%) present in the medium (see W. F. Hink, 226 Nature (London) 466-467 (1970)) were required for the expression of IFN-$\beta$ in S. frugiperda cells infected with Ac380-IFN-$\beta$. At 8 hours post infection, the medium was replaced with Grace's medium (no serum albumin, see T. C. C. Grace, 195 Nature (London) 788-789 (1962)) containing 0 to 10% fetal bovine serum. Each of the modified media were assayed for interferon activity at 48 hours post infection. Without serum, there was about a 10 fold reduction in interferon activity. With the addition of 0.5% serum, the same level of activity was produced as in controls containing 10% serum. The specific activity of IFN-$\beta$ in Ac380-IFN-$\beta$ infected cells was about 5 x $10^6$ IU/mg of protein when produced in Grace's medium containing 0.5% serum. Assuming that the activity of purified $\beta$-interferon is 2 x $10^8$ IU/ml (see E. Knight, Jr., 73 Proc. Natl. Acad. Sci. U.S.A. 520-523 (1976)), $\beta$-interferon would represent about 1% of the total protein in the medium.

Further analysis of the interferon activity measured in the medium of Ac373-IFN-$\beta$ and Ac380-IFN-$\beta$ infectedcells revealed two polypeptides of 17,000 (17K) and 20.5K molecular weight. The sizes of nonglycosylated and glycosylated human IFN-$\beta$ proteins are reported to be comparable to the 17K and 20.5K polypeptides, respectively. At 30 hours post infection the 17K polypeptide was being made in Ac360-IFN-$\beta$-infectec cells, and by 48 hours post infection both 17K and 20.5K polypeptides were detected. Only the 17K polypeptide could be detected in Ac311-IFN-$\beta$-infected cells at 30 and 48 hours post infection. An abundantly produced 23.5K protein was observed in Ac360-IFN-$\beta$ infected cells. This size is expected for a hybrid protein consisting of the entire interferon protein, including the 21 amino acid signal peptide plus an additional 14 amino acids derived from the first 10 codons of the polyhedrin gene and the BamHI linker sequences.

The 17K and 20.5K proteins made in Ac380-IFN-$\beta$ and, to a lesser extent, Ac360-IFN-$\beta$-infected cells reacted with human IFN-$\beta$ monoclonal antibody. (The IFN-$\beta$ monoclonal antibody was provided by P. W. Trown and Hoffmann - La Roche Inc.) A reduced reaction of this antibody to 20.5K protein as compared with that to the 17K protein was noted. This was in part due to the fact that 17K accumulates to higher levels in cells than does 20.5K. In addition, the antibody may be reacting to an epitope on the 17K polypeptide that is partially masked by, for example, glycosylation of the 20.5K polypeptide.

The putative hybrid 23.5K and 32K proteins also reacted with IFN-$\beta$ antibody. Polyclonal antibody to polyhedrin recognized the 10 amino acids of the 23.5K protein and the 57 amino acids of the 32K protein that would be predicted from the DNA sequence to be present at the N-terminal ends of the hybrid proteins.

To demonstrate that 20.5K IFN- was glycosylated, Ac380-IFN-$\beta$-infected cells were labeled late in infection with [$^3$H] mannose. The 20.5K IFN- and three additional proteins were the major mannose-containing glycoproteins labeled in Ac380-IFN-$\beta$-infected cells.

EXAMPLE VII

CONSTRUCTION OF CHLORAMPHENICOL ACETYLTRANSFERASE GENE AND AcMNPV RECOMBINANT TRANSFER VECTORS

The E. coli transposable element Tn9 contains the gene for the enzyme chloramphenicol acetyltransferase (CAT), which confers resistance to the antibiotic chloramphenicol. Expression of CAT in eucaryotic vectors has been shown to be a convenient means of measuring the expression of promoters in animal cells (see Mackett, M., G. Smith, and B. Moss. 1984. J. Virol. 49:857-864 and references therein). The AcMNPV-CAT expression vectors are useful in experiments designed to optimize the production of foreign genes in baculovirus vectors.

A 770 base pair TaqI DNA fragment containing the CAT-coding sequence was isolated from pBR328 and cloned into the AccI site of pUC7. The CAT-coding sequence was excised with BamHI and inserted into the BamHI site in pAc373. The resulting plasmid transfer vector is called pAc373-CAT.

EXAMPLE VIII

CONSTRUCTION OF A HUMAN INTERLEUKIN-2 GENE AND AcMNPV RECOMBINANT TRANSFER VECTOR

Human interleukin-2 (IL2) is produced in minute quantities in human lymphocytes that have been stimulated by mitogens or antigens. IL-2 was originally described as a factor capable of maintaining long-term growth of T lymphocyte cells in culture (Morgan, D.A., Ruscetti, F.W. and Gallo, R., Science 193:1007-1008 (1976)). It appears to play a central role in the stimulation and maintenance of immune responses and has been implicated as being involved in certain human diseases (Altman, A., Theofilopoulos, A.N., Weiner, R., Katz, D.H. and Dixon, F.J., J. Expl. Med. 154:1403-1417 (1981)). The use of AcMNPV expression vectors for the production of large quantities of IL-2 is expected to greatly facilitate the clinical diagnostic and therapeutic manipulation of the human immune system. Recently, several laboratories have reported the isolation of the gene for IL2 and the production of biologically active IL-2 in bacterial cells using plasmid vectors (see Rosenberg, et al. Science 223:1412-1415 (1984) and references therein).

A 1000 base pair BamHI fragment containing the IL2-coding sequences was isolated from pIL2-2B, and inserted into the BamHI site in pAc373 and pAc380. The resulting plasmid transfer vectors are called pAc373-IL2 and pAc380-IL2.

EXAMPLE IX

TRANSFER OF THE POLYHEDRIN-IL2 AND CAT GENES TO AcMNPV GENOME

The transfer of the polyhedrin-IL2 and CAT genes into the AcMNPV genome and selection of recombinant AcMNPV expression vectors was done as described above in EXAMPLE IV. The resulting AcMNPV expression vectors produced from pAc373-IL2, pAc380-IL2, and pAc373-CAT are called Ac373-IL2, Ac380-IL2, and Ac373-CAT, respectively.

EXAMPLE X

PRODUCTION OF IL2 USING AcMNPV EXPRESSION VECTORS

S. frugiperda cells were infected with Ac373-IL2 or Ac383-IL2 expression vectors as described for Ac380-IFN-$\beta$. At 48 hours post infection the media and infected cells were collected and the levels of interleukin biological activity was measured using the IL2 assay described by Gillis, et al. J. Immunol. 120:2027-2032 (1978). Using this assay the specific activity of IL2 has been determined to be $1 \times 10^8$ units per milligram of protein. Both expression vectors produced high levels of interleukin activity, but Ac373-IL2 produced approximately four times more interleukin than Ac380-IL2 (see Table 2). About 80% of the interleukin activity was present in the media, demonstrating that the protein is being efficiently secreted from the cells during infection. In a separate experiment conducted by Dr. Grace Ju, Hoffmann-La Roche Research Center, S. frugiperda cells were infected with Ac373-IL2 and Ac380-IL2 and the levels of interleukin activity were measured in the media at 24 hours, 48 hours, and 72 hours post infection. Fresh media were applied at 24 hours and 48 hours. Virtually all the activity was produced between 0 to 24 hours and 24 to 48 hours post infection (Table 2). From the specific activity of IL2, at least 1 mg of IL2 protein is calculated to be produced and secreted per liter of Ac373-IL2 infected cells.

An analysis of the proteins being synthesized in Ac373-IL2 and Ac380-IL2 infected cells was conducted. There are two proteins being made in abundance by these AcMNPV expression vectors that are not made in AcMNPV infected cells. These new proteins are about 15.5K and 16K daltons in size. This is consistent with the fact that the size of interleukin predicted from the DNA sequence is about 15.5K (assuming that the 20 amino acid signal peptide at the amino-terminal end of the protein is removed).

16

Table 2

The production of interleukin-2 activity in
S. frugiperda cells infected with AcMNPV expression
vectors.  In Infection No. 1 samples of the media and
infected cells were collected at 48 hours post infection.
In Infection No. 2 samples of the media were collected at
24 hours, 48 hours, and 72 hours post infection and fresh
media were applied at 24 hours and 48 hours post
infection.

INFECTION NO. 1[a]

| Vector | Cells (Units/l) | Media (Units/l) | Total mg/l | %Activity Secreted |
|--------|------------------|------------------|------------|---------------------|
| Ac373-IL2 | $2.5 \times 10^7$ | $1 \times 10^8$ | 1.25 | 80% |
| Ac380-IL2 | $1.0 \times 10^7$ | $5 \times 10^7$ | 0.6 | 84% |

INFECTION NO. 2[b]

| Vector | Hours p.i. | Media (Units/l) | Total Mg/l |
|--------|------------|------------------|------------|
| Ac373-IL2 | 24 h | $5.1 \times 10^7$ | 0.5 |
| AC373-IL2 | 48 h | $5.1 \times 10^7$ | 0.5 |
| Ac373-IL2 | 72 h | $4.8 \times 10^6$ | 0.05 |
| Ac380-IL2 | 24 h | $1.3 \times 10^7$ | 0.13 |
| Ac380-IL2 | 48 h | $1.3 \times 10^7$ | 0.13 |
| Ac380-IL2 | 72 h | $6.4 \times 10^6$ | 0.06 |

[a]   Produced at Texas A & M University

[b]   Produced at Hoffmann-La Roche Research Center


EXAMPLE XI

PRODUCTION OF CAT USING AcMNPV EXPRESSION VECTORS

S. frugiperda cells were infected with Ac373-CAT as described for Ac380-IFN-$\beta$ and at 24 hours post infection CAT enzyme activity was measured in the cells and medium as described by Mackett, et al. IBID. There was no detectable CAT enzyme activity present in uninfected cells or cells infected with AcMNPV; however, high levels of activity were produced by Ac373-CAT in both the cells and medium.

An analysis of the proteins synthesized in Ac373-CAT infected cells was conducted. A new protein of 27K daltons was produced by this expression vector that was not made in AcMNPV infected cells. The size of CAT can be predicted from the DNA sequence to be about 27K. An abundance of the 27K protein was present in both the infected cells and medium. From the amount of protein that was observed on polyacrylamide gels, about 40 mg of CAT enzyme is estimated to be produced per liter of Ac373-CAT infected cells.

The invention and the advantages and opportunities presented by it will be recognized from the above description, which merely describes several preferred embodiments of the invention. It is apparent that many changes in the materials, methods and amounts of materials utilized may be made without departing from the scope and spirit of the invention or compromising any of its advantages. Further, it will be recognized that the above-descibed invention has uses which are predicated on making advantageous use of the fact that the present invention may be utilized to insert any selected gene at any of several locations in a baculovirus genome.

For instance, the fact that procedures are available for the isolation of recombinant AcMNPV viruses in which all or a portion of the polyhedrin gene has not been deleted makes it possible to utilize the present invention in a number of ways. These uses may take advantage of the fact that the polyhedrin coating of the occluded form of the AcMNPV virus is so resistant to external influences. For instance, as discussed in Example III, a selected gene could be cloned into the viral genome at a location other than in the polyhedrin gene, in particular at a location such that expression of the selected gene would be controlled by the 10K promoter so that it would be expressed at high levels. This recombinant AcMNPV virus could then be isolated and utilized as a stable expression vector.

Such a stable expression vector can be utilized as a stable form in which a recombinant AcMNPV virus could be transferred, along with a culture of the appropriate host cells and sufficient media, from one laboratory to another for eventual use in the production of a desired protein at some designated time in the future.

The expression vector might also be used in a system for controlling pest insect populations by selecting a gene which produces a protein which is toxic to a specific host insect species or a broad spectrum of susceptible host insect species and cloning that gene into the AcMNPV expression vector (these possibilities are discussed by L.K. Miller, et al., 219 Science 715-721 (1983)). The recombinant expression vector could be applied to the plant or animal upon which the insect is a pest, and when it is ingested by the pest insect, as discussed above, the occlusion will dissociate in the lumen of the intestine, the recombinant virus will invade the cells of the intestinal wall and begin replication.

During replication, the gene for the protein toxic to the pest insect will be expressed, resulting in the disabilitation or death of the insect in a much shorter period than if the insect had ingested the wild type AcMNPV virus, in which case the insect would by lysed after a period which would vary depending upon the extent of the viral infection. Indications from experiments in this and other laboratories are that expression of the 10K protein occurs as early as 24 hours post infection and at high levels at about 48 hours. Consequently, a gene encoding for a desired insect toxin which is cloned into the AcMNPV genome under the control of the 10K promoter would also be expected to be expressed according to that time schedule. The death or disabilitation of the insect could be expected soon after the initiation of expression of that selected gene, resulting in a concomitant decrease in damage to the plants or animals upon which that pest insect preys as compared to an infection of the insect with the wild-type baculovirus.

The gene could also be inserted into the baculovirus genome so that it was fused to the polyhedrin structural sequence in such a way that when the polyhedrin coating is dissociated by the alkaline conditions of the insect gut, the toxic gene product would be released. Such a use of the present invention would result in the poisoning of the insect without expression of the recombinant gene in the insect intestinal cells.

Further, it will be recognized that even higher levels of gene expression than those measured in the above-described examples are possible utilizing the present invention. For instance, the IFN-$\beta$ gene (or any other gene) could be cloned into the baculovirus genome more than once. In particular, copies could be inserted so that expression is under control of the polyhedrin promoter, other copies could be inserted so that expression is under control of the 10K promoter, and then several more copies could be inserted at various other restriction sites, each copy including either its own promoter or some other DNA sequence recognized by the baculovirus as a promoter. Upon infection into susceptible insect cells, the amount of interferon (or other polypeptide) produced could be vastly increased over the levels measured above.

Further modifications of the invention herein disclosed will occur to persons skilled in the art who have the benefit of this disclosure, and all such modifications are deemed to be within the spirit and scope of the invention as defined by the appended claims.

**Claims**

**Claims for the following Contracting States : BE, CH, DE, DK, ES, FR, GB, GR, IT, LI, LU, NL, SE**

1. A method for producing a recombinant baculovirus expression vector, capable of expressing a selected non-fused recombinant heterologous gene in a host insect cell, characterized in that it comprises:

   (a) cleaving baculovirus DNA to produce a DNA fragment comprising a baculovirus polyhedrin gene having sufficient homology with a nucleotide sequence as illustrated in Fig. 3 and sufficient flanking baculovirus DNA sequences to facilitate homologous recombination;

   (b) inserting said baculovirus DNA fragment into a cloning vehicle to form a baculovirus gene transfer vector;

   (c) creating a unique restriction site for cloning a selected heterologous gene, said unique restriction site being located within the polyhedrin gene;

   (d) deleting all of the sequences from said baculovirus DNA fragment encoding for polyhedrin protein synthesis, and if desired, further deleting some of the sequences from said baculovirus DNA fragment upstream of the polyhedrin ATG translation initiation start site, such that said unique restriction site is located within the polyhedrin gene at a position ranging from the polyhedrin transcriptional start signal to the ATG translation initiation start site;

   (e) preparing a recombinant baculovirus transfer vector by inserting at least one selected heterologous gene into the unique restriction site such that said selected gene is under the transcriptional control of the baculovirus polyhedrin promoter;

   (f) contacting said recombinant baculovirus transfer vector with baculovirus DNA so as to effect recombination, thereby producing a mixture of recombinant and nonrecombinant baculoviruses; and

   (g) isolating a recombinant baculovirus expression vector from said mixture.

2. A method of producing a baculovirus recombinant transfer vector, having at least one selected heterologous gene, characterized in that it comprises:

   (a) cleaving baculovirus DNA to produce a DNA fragment comprising a baculovirus polyhedrin gene having sufficient homology with a nucleotide sequence as illustrated in Fig. 3 and sufficient flanking baculovirus DNA sequences to facilitate homologous recombination;

   (b) inserting said baculovirus DNA fragment into a cloning vehicle to form a baculovirus gene transfer vector;

   (c) creating a unique restriction site for cloning a selected heterologous gene, said unique restriction site being located within the polyhedrin gene;

   (d) deleting all of the sequences from said baculovirus DNA fragment encoding for polyhedrin protein synthesis, and if desired, further deleting some of the sequences from said baculovirus DNA fragment upstream of the polyhedrin ATG translation initiation start site, such that said unique restriction site is located within the polyhedrin gene at a position ranging from the polyhedrin transcriptional start signal to the ATG translation initiation start site; and

   (e) inserting at least one selected heterologous gene into the unique restriction site such that said selected gene is under the transcriptional control of the baculovirus polyhedrin promoter.

3. A method for producing a baculovirus transfer vector, having a unique restriction site under the transcriptional control of the polyhedrin promoter, characterized in that it comprises:

   - cleaving baculovirus DNA to produce a DNA fragment containing at least a baculovirus polyhedrin gene having sufficient homology with a nucleotide sequence as illustrated in Fig. 3 and sufficient flanking baculovirus DNA sequences to facilitate homologous recombination;

   - inserting said DNA fragment into a cloning vehicle so as to produce a baculovirus gene transfer vector;

   - creating a unique restriction site for cloning a selected heterologous gene, said unique restriction site being located within the polyhedrin gene; and

   - deleting all of the sequences from said baculovirus DNA fragment encoding for polyhedrin protein synthesis, and if desired, further deleting some of the sequences from said baculovirus DNA fragment upstream of the polyhedrin ATG translation initiation start site, such that said unique restriction site is located within the polyhedrin gene at a position ranging from the polyhedrin transcriptional start signal to the ATG translation initiation start site.

4. A method for synthesizing a selected nonfused heterologous polypeptide characterized in that it comprises infecting a susceptible host insect cell with a recombinant baculovirus expression vector comprising at least one selected heterologous gene inserted into a baculovirus polyhedrin gene between the polyhedrin transcriptional start signal and the polyhedrin ATG translation initiation start site, and under the transcriptional control of a baculovirus polyhedrin promoter having sufficient homology with a nucleotide sequence as illustrated in Fig. 3.

5. A method according to anyone of the claims 1 to 3 wherein the unique restriction site is a BamHI site.

6. A method according to anyone of the claims 1 to 3 wherein said unique restriction site is located at a position ranging from nucleotide -1 to nucleotide - 10 of said baculovirus polyhedrin gene.

7. A method according to anyone of the claims 1 to 4 wherein the baculovirus polyhedrin gene is derived from Autographa californica MNPV, Trichoplusia ni MNPV, Rachiplusia ou MNPV, or Galleria mellonella MNPV.

8. A method according to anyone of claims 1 to 4 wherein the baculovirus gene is derived from Autographa californica MNPV.

9. A method according to claim 4 wherein the selected heterologous gene is inserted at a BamHI site.

10. A recombinant baculovirus expression vector, capable of expressing a selected heterologous gene in a host insect cell, characterized in that it comprises at least one selected heterologous gene inserted into a baculovirus polyhedrin gene at a position ranging from the polyhedrin transcriptional start signal to the polyhedrin ATG translation initiation start site, and under the transcriptional control of a baculovirus polyhedrin promoter having sufficient homology with a nucleotide sequence as illustrated in Fig. 3.

11. A recombinant transfer vector, capable of introducing a selected heterologous gene into a baculovirus genome, characterized in that it comprises a cloning vehicle having a DNA sequence comprising a baculovirus polyhedrin gene and at least one selected heterologous gene linked to said baculovirus polyhedrin gene at a position ranging from the polyhedrin transcriptional start signal to the polyhedrin ATG translation initiation start site and under the transcriptional control of a promoter of said baculovirus polyhedrin gene, said baculovirus polyhedrin promoter having sufficient homology with a nucleotide sequence as illustrated in Fig. 3, and sufficient flanking baculovirus DNA sequences to facilitate homologous recombination.

12. A baculovirus transfer vector, characterized in that it comprises a baculovirus polyhedrin promoter having sufficient homology with a nucleotide sequence as illustrated in Fig. 3, and sufficient flanking baculovirus DNA sequences to facilitate homologous recombination, and a unique restriction site for cloning a selected heterologous gene, said unique restriction site being located within the polyhedrin gene at a position ranging from the polyhedrin transcriptional start signal to the polyhedrin ATG translation initiation start site.

13. A recombinant baculovirus expression vector, capable of producing a selected nonfused heterologous protein in a host insect cell, wherein said expression vector is a baculovirus genome comprising a selected heterologous gene including its own ATG signal and under the transcriptional control of a baculovirus polyhedrin promoter having sufficient homology with a nucleotide sequence as illustrated in Fig. 3.

14. A vector according to anyone of the claims 10 to 13 wherein the baculovirus promoter is derived from Autographa californica MNPV, Trichoplusia ni MNPV, Rachiplusia ou MNPV or Galleria mellonella MNPV.

15. A vector according to anyone of the claims 10 to 13 wherein the baculovirus promoter is derived from Autographa californica MNPV.

16. A vector according to anyone of the claims 10 to 13 wherein the selected heterologous gene is inserted at a BamHI site.

**17.** A vector according to claim 12 wherein the unique restriction site is a BamHI site.

**18.** A vector according to anyone of the claims 10 or 11 wherein the selected heterologous gene is introduced at a position ranging from nucleotide -1 to nucleotide - 10 of said baculovirus polyhedrin gene.

**19.** A vector according to claim 12 wherein said unique restriction site is located at a position ranging from nucleotide -1 to nucleotide - 10 of said baculovirus polyhedrin gene.

**20.** The recombinant baculovirus expression vector of claim 13 wherein all or a portion of the DNA sequence coding for the baculovirus polyhedrin structural gene under the transcriptional control of the promoter is deleted.

**21.** The baculovirus transfer vector of claim 12 which is designated as NRRL B-15428 or NRRL B-15778 on deposit with the Agricultural Research Culture Collection.

**Claims for the following Contracting State : AT**

**1.** A method for producing a recombinant baculovirus expression vector, capable of expressing a selected non-fused recombinant heterologous gene in a host insect cell, characterized in that it comprises:
a) cleaving baculovirus DNA to produce a DNA fragment comprising a baculovirus polyhedrin gene having sufficient homology with a nucleotide sequence as illustrated in Fig. 3 and sufficient flanking baculovirus DNA sequences to facilitate homologous recombination;
b) inserting said baculovirus DNA fragment into a cloning vehicle to form a baculovirus gene transfer vector;
c) creating a unique restriction site for cloning a selected heterologous gene, said unique restriction site being located within the polyhedrin gene;
d) deleting all of the sequences from said baculovirus DNA fragment encoding for polyhedrin protein synthesis, and if desired, further deleting some of the sequences from said baculovirus DNA fragment upstream of the polyhedrin ATG translation initiation start site, such that said unique restriction site is located within the polyhedrin gene at a position ranging from the polyhedrin transcriptional start signal to the ATG translation initiation start site;
e) preparing a recombinant baculovirus transfer vector by inserting at least one selected heterologous gene into the unique restriction site such that said selected gene is under the transcriptional control of the baculovirus polyhedrin promoter;
f) contacting said recombinant baculovirus transfer vector with baculovirus DNA so as to effect recombination, thereby producing a mixture of recombinant and nonrecombinant baculoviruses; and
g) isolating a recombinant baculovirus expression vector from said mixture.

**2.** A method of producing a baculovirus recombinant transfer vector, having at least one selected heterologous gene characterized in that it comprises:
a) cleaving baculovirus DNA to produce a DNA fragment comprising a baculovirus polyhedrin gene having sufficient homology with a nucleotide sequence as illustrated in Fig. 3 and sufficient flanking baculovirus DNA sequences to facilitate homologous recombination;
b) inserting said baculovirus DNA fragment into a cloning vehicle to form a baculovirus gene transfer vector;
c) creating a unique restriction site for cloning a selected heterologous gene, said unique restriction site being located within the polyhedrin gene;
d) deleting all of the sequences from said baculovirus DNA fragment encoding for polyhedrin protein synthesis, and if desired, further deleting some of the sequences from said baculovirus DNA fragment upstream of the polyhedrin ATG translation initiation start site such that said unique restriction site is located within the polyhedrin gene at a position ranging from the polyhedrin transcriptional start signal to the ATG translation initiation start site; and
e) inserting at least one selected heterologous gene into the unique restriction site such that said selected gene is under the transcriptional control of the baculovirus polyhedrin promoter.

**3.** A method for producing a baculovirus transfer vector, having a unique restriction site under the transcriptional control of the polyhedrin promoter, characterized in that it comprises:

- cleaving baculovirus DNA to produce a DNA fragment containing at least a baculovirus polyhedrin gene having sufficient homology with a nucleotide sequence as illustrated in Fig. 3 and sufficient flanking baculovirus DNA sequences to facilitate homologous recombination;
- inserting said DNA fragment into a cloning vehicle so as to produce a baculovirus gene transfer vector;
- creating a unique restriction site for cloning a selected heterologous gene, said unique restriction site being located within the polyhedrin gene; and
- deleting all of the sequences from said baculovirus DNA fragment encoding for polyhedrin protein synthesis, and if desired, further deleting some of the sequences from said baculovirus DNA fragment upstream of the polyhedrin ATG translation initiation start site, such that said unique restriction site is located within the polyhedrin gene at a position ranging from the polyhedrin transcriptional start signal to the ATG translation initiation start site.

4. A method for synthesizing a selected nonfused heterologous polypeptide characterized in that it comprises infecting a susceptible host insect cell with a recombinant baculovirus expression vector comprising at least one selected heterologous gene inserted into a baculovirus polyhedrin gene between the polyhedrin transcriptional start signal and the polyhedrin ATG translation initiation start site, and under the transcriptional control of a baculovirus polyhedrin promoter having sufficient homology with a nucleotide sequence as illustrated in Fig. 3.

5. A method according to anyone of the claims 1 to 3 wherein the unique restriction site is a BamHI site.

6. A method according to anyone of the claims 1 to 3 wherein said unique restriction site is located at a position ranging from nucleotide -1 to nucleotide -10 of said baculovirus polyhedrin gene.

7. A method according to anyone of the claims 1 to 4 wherein the baculovirus polyhedrin gene is derived from Autographa californica MNPV, Trichoplusia ni MNPV, Rachiplusia ou MNPV or Galleria mellonella MNPV.

8. A method according to anyone of the claims 1 to 4 wherein the baculovirus polyhedrin gene is derived from Autographa californica MNPV.

9. A method according to claim 4 wherein the selected heterologous gene is inserted at a BamHI site.

10. A method according to anyone of the claims 1 to 9 for preparing transfer vectors designated as NRRL B-15428 or NRRL B-15778 on deposit with the Agricultural Research Culture Collection.

**Revendications**

**Revendications pour les Etats contractants suivants : BE, CH, DE, DK, ES, FR, GB, GR, IT, LI, LU, NL, SE**

1. Procédé pour la préparation d'un vecteur d'expression de baculovirus, capable d'exprimer un gène hétérologue recombinant non fusionné sélectionné dans une cellule hôte d'insecte, caractérisé en ce qu'il comprend :

(a) la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;

(b) l'insertion de ce fragment d'ADN de baculovirus dans un véhicule de clonage pour former un vecteur de transfert de gène de baculovirus;

(c) la création d'un site de restriction unique pour le clonage d'un gêne hétérologue sélectionné, ce site de restriction unique étant situé à l'intérieur du gène de la polyhédrine;

(d) la délétion de toutes les séquence de ce fragment d'ADN de baculovirus codant pour la synthèse de la protéine de polyhédrine et si cela est désiré, la délétion supplémentaire de certaines des séquences de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG;

(e) la préparation d'un vecteur recombinant de transfert de baculovirus par insertion d'au moins un gène hétérologue sélectionné dans le site de restriction unique de telle sorte que ce gène sélectionné se trouve sous le contrôle transcriptionnel du promoteur de la polyhédrine du baculovirus;

(f) la mise en contact de ce vecteur recombinant de transfert de baculovirus avec un ADN de baculovirus pour effectuer une recombinaison, afin de produire ainsi un mélange de baculovirus recombinant et de baculovirus non recombinant; et

(g) l'isolation d'un vecteur recombinant d'expression de baculovirus à partir de ce mélange.

2. Procédé pour la préparation d'un vecteur recombinant de transfert de baculovirus, ayant au moins un gène hétérologue sélectionné, caractérisé en ce qu'il comprend :

(a) la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;

(b) l'insertion de ce fragment d'ADN de baculovirus dans un véhicule de clonage pour former un vecteur de transfert de gène de baculovirus;

(c) la création d'un site de restriction unique pour le clonage d'un gène hétérologue sélectionné, ce site de restriction unique étant situé à l'intérieur du gène de la polyhédrine;

(d) la délétion de toutes les séquences de ce fragment d'ADN de baculovirus codant pour la synthèse de la protéine de polyhédrine et si cela est désiré, la délétion supplémentaire de certaines des séquences de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG;

(e) l'insertion d'un moins un gène hétérologue sélectioné dans le site de restriction unique de telle sorte que ce gène sélectionné se trouve sous le contrôle transcriptionnel du promoteur de la polyhédrine du baculovirus.

3. Procédé pour la préparation d'un vecteur de transfert de baculovirus, ayant un site de restriction unique sous le contrôle transcriptionnel du promoteur de la polyhédrine, caractérisé en ce qu'il comprend :

- la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;

- l'insertion de ce fragment d'ADN de baculovirus dans un véhicule de clonage pour former un vecteur de transfert de gène de baculovirus;

- la création d'un site de restriction unique pour le clonage d'un gène hétérologue sélectionné, ce site de restriction unique étant situé à l'intérieur du gène de la polyhédrine;

- la délétion de toutes les séquences de ce fragment d'ADN de baculovirus codant pour la synthèse de la protéine de polyhédrine et si cela est désiré, la délétion supplémentaire de certaines des séquence de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG.

4. Procédé pour synthétiser un polypeptide hétérologue non fusionné sélectionné, caractérisé en ce qu'il comprend l'infection d'une cellule hôte d'insecte sensible avec un vecteur recombinant d'expression de baculovirus comprenant au moins un gène hétérologue sélectionné inséré dans un gène de la polyhédrine de baculovirus entre le signal départ de la transcription de la polyhédrine et le site départ de l'initiation de la traduction ATG de la polyhédrine, et sous le contrôle transcriptionnel d'un promoteur de la polyhédrine de baculovirus ayant une homologie suffisante avec une séquence de nucléotides telle qu'illustrée dans la figure 3.

5. Procédé suivant l'une quelconque des revendications 1 à 3, dans lequel le site de restriction unique est un site Bam HI.

6. Procédé suivant l'une quelconque des revendications 1 à 3, dans lequel ce site de restriction unique est situé à une position comprise entre le nucléotide -1 et le nucléotide -10 de ce gène de la polyhédrine de baculovirus.

7. Procédé suivant l'une quelconque des revendications 1 à 4, dans lequel le gène de la polyhédrine de baculovirus est dérivé d'Autographa californica MNPV, de Trichoplusia ni MNPV, de Rachiplusia ou MNPV ou de Galleria mellonella MNPV.

8. Procédé suivant l'une quelconque des revendications 1 à 4, dans lequel le gène de baculovirus est dérivé d'Autographa californica MNPV.

9. Procédé suivant la revendication 4, dans lequel le gène hétérologue sélectionné est inséré au niveau du site Bam HI.

10. Vecteur recombinant d'expression de baculovirus capable d'exprimer un gène hétérologue sélectionné dans une cellule hôte d'insecte, caractérisé en ce qu'il comprend au moins un gène hétérogue sélectionné inséré dans un gène de la polyhédrine de baculovirus à une position comprise entre le signal départ de la transcription de la polyhédrine et le site départ de l'initiation de la traduction ATG de la polyhédrine, et sous le contrôle transcriptionnel d'un promoteur de la polyhédrine de baculovirus ayant une homologie suffisante avec une séquence de nucléotides telle qu'illustrée dans la figure 3.

11. Vecteur recombinant de transfert, capable d'introduire un gène hétérologue sélectionné dans un génome de baculovirus, caractérisé en ce qu'il comprend un véhicule de clonage ayant une séquence d'ADN comprenant un gène de la polyhédrine de baculovirus et au moins un gène hétérologue sélectionné lié à ce gène de la polyhédrine de baculovirus à une position comprise entre le signal départ de la transcription de la polyhédrine et le site départ de l'initiation de la traduction ATG de la polyhédrine et sous le contrôle transcriptionnel d'un promoteur de ce gène de la polyhédrine de baculovirus, ce promoteur de la polyhédrine de baculovirus ayant une homologie suffisante avec une séquence nucléotidique telle qu'illustrée dans la figure 3, et flanqué de séquences suffisantes d'ADN de baculovirus pour faciliter une recombinaison homologue.

12. Vecteur de transfert de baculovirus, caractérisé en ce qu'il comprend un promoteur de la polyhédrine de baculovirus ayant une homologie suffisante avec une séquence nucléotidique telle qu'illustrée dans la figure 3, et flanqué de séquences suffisantes d'ADN de baculovirus pour faciliter une recombinaison homologue, et un site de restriction unique pour le clonage d'un gène hétérologue sélectionné, ce site de restriction unique étant situé à l'intérieur du gène de lapolyhédrine a une position comprise entre le signal départ de la transcription de la polyhédrine et le site départ de l'initiation de la traduction ATG de la polyhédrine.

13. Vecteur recombinant d'expression de baculovirus, capable de produire une protéine hétérologue non fusionnée sélectionnée dans une cellule hôte d'insecte dans lequel ce vecteur d'expression est un génome de baculovirus comprenant un gène hétérologue sélectionné comprenant son propre signal ATG et sous le contrôle transcriptionnel d'un promoteur de la polyhédrine de baculovirus ayant une homologie suffisante avec une séquence de nucléotides telle qu'illustrée dans la figure 3.

14. Vecteur suivant l'une quelconque des revendications 10 à 13, dans lequel le promoteur de baculovirus est dérivé d'Autographa californica MNPV, de Trichoplusia ni MNPV, de Rachiplusia ou MNPV ou de Galleria mellonella MNPV.

15. Vecteur suivant l'une quelconque des revendications 10 à 13, dans lequel le promoteur de baculovirus est dérivé d'Autographa californica MNPV.

16. Vecteur suivant l'une quelconque des revendications 10 à 13, dans lequel le gène hétérologue sélectionné est inséré au niveau du site Bam HI.

17. Vecteur suivant la revendication 12, dans lequel le site de restriction unique est un site Bam HI.

18. Vecteur suivant l'une quelconque des revendications 10 ou 11, dans lequel le gène hétérologue sélectionné est introduit à une position comprise entre le nucléotide - 1 et le nucléotide - 10 de ce

24

gène de la polyhédrine de baculovirus.

19. Vecteur suivant la revendication 12, dans lequel ce site de restriction unique est situé à une position comprise entre le nucléotide -1 et le nucléotide -10 de ce gène de la polyhédrine de baculovirus.

20. Vecteur recombinant d'expression de baculovirus suivant la revendication 13, dans lequel la totalité ou une partie de la séquence d'ADN codant pour le gène structural de la polyhédrine de baculovirus sous le contrôle transcriptionnel du promoteur, est éliminée.

21. Vecteur de transfert de baculovirus suivant la revendication 12, qui est désigné sous le nom de NRRL B-15428 ou de NRRL B-15778 lors du dépôt à la Agricultural Research Culture Collection.

**Revendications pour l'Etat contractant suivant : AT**

1. Procédé pour la préparation d'un vecteur d'expression de baculovirus, capable d'exprimer un gène hétérologue recombinant non fusionné sélectionné dans une cellule hôte d'insecte, caractérisé en ce qu'il comprend:

   (a) la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;

   (b) l'insertion de ce fragment d'ADN de baculovirus dans un véhicule de clonage pour former un vecteur de transfert de gène de baculovirus;

   (c) la création d'un site de restriction unique pour le clonage d'un gène hétérologue sélectionné, ce site de restriction unique étant situé à l'intérieur du gène de la polyhédrine;

   (d) la délétion de toutes les séquences de ce fragment d'ADN de baculovirus codant pour la synthèse de la protéine de polyhédrine et si cela est désiré, la déletion supplémentaire de certaines des séquences de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG;

   (e) la préparation d'un vecteur recombinant de transfert de baculovirus par insertion d'au moins un gène hétérologue sélectionné dans le site de restriction unique de telle sorte que ce gène sélectionné se trouve sous le contrôle transcriptionnel du promoteur de la polyhédrine du baculovirus;

   (f) la mise en contact de ce vecteur recombinant de transfert de baculovirus avec un ADN de baculovirus pour effectuer une recombinaison, afin de produire ainsi un mélange de baculovirus recombinant et de baculovirus non recombinant; et

   (g) l'isolation d'un vecteur recombinant d'expression de baculovirus à partir de ce mélange.

2. Procédé pour la préparation d'un vecteur recombinant de transfert de baculovirus, ayant au moins un gène hétérologue sélectionné, caractérisé en ce qu'il comprend :

   (a) la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;

   (b) l'insertion de ce fragment d'ADN de baculovirus dans un véhicule de clonage pour former un vecteur de transfert de gène de baculovirus;

   (c) la création d'un site de restriction unique pour le clonage d'un gène hétérologue sélectionné, ce site de restriction unique étant situé à l'intérieur du gène de la polyhédrine;

   (d) la délétion de toutes les séquences de ce fragment d'ADN de baculovirus codant pour la synthèse de la protéine de polyhédrine et si cela est désiré, la délétion supplémentaire de certaines des séquences de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG;

   (e) l'insertion d'un moins un gène hétérologue sélectionné dans le site de restriction unique de telle sorte que ce gène sélectionné se trouve sous le contrôle transcriptionnel du promoteur de la polyhédrine du baculovirus.

3. Procédé pour la préparation d'un vecteur de transfert de baculovirus, ayant un site de restriciton unique sous le contrôle transcriptionnel du promoteur de la polyhédrine, caractérisé en ce qu'il comprend :

- la coupure d'un ADN de baculovirus pour produire un fragment d'ADN comprenant un gène de la polyhédrine de baculovirus ayant une homologie suffisante avec une séquence de nucléotides telle qu'illustrée dans la figure 3 et flanqué de séquences suffisantes d'ADN de baculovirus pour faciliter une recombinaison homologue;
- l'insertion de ce fragment d'ADN de baculovirus dans un véhicule de clonage pour former un vecteur de transfert de gène de baculovirus;
- la création d'un site de restriction unique pour le clonage d'un gène hétérologue sélectionné, ce site de restriction unique étant situé à l'intérieur du gène de la polyhédrine;
- la délétion de toutes les séquences de ce fragment d'ADN de baculovirus codant pour la synthèse de la protéine de polyhédrine et si cela est désiré, la délétion supplémentaire de certaines des séquences de ce fragment d'ADN de baculovirus en amont du site de départ de l'initiation de traduction ATG de la polyhédrine, de telle sorte que ce site de restriction unique se trouve à l'intérieur du gène de la polyhédrine à une position comprise entre le signal départ de la transcription de la polyhédrine et le site de départ de l'initiation de la traduction ATG.

4. Procédé pour synthétiser un polypeptide hétérologue non fusionné sélectionné, caractérisé en ce qu'il comprend l'infection d'une cellule hôte d'insecte sensible avec un vecteur recombinant d'expression de baculovirus comprenant au moins un gène hétérologue sélectionné inséré dans un gène de la polyhédrine de baculovirus entre le signal départ de la transcription de la polyhédrine et le site départ de l'initiation de la traduction ATG de la polyhédrine, et sous le contrôle transcriptionnel d'un promoteur de la polyhédrine de baculovirus ayant une homologie suffisante avec une séquence de nucléotides telle qu'illustrée dans la figure 3.

5. Procédé suivant l'une quelconque des revendications 1 à 3 dans lequel le site de restriction unique est un site Bam HI.

6. Procédé suivant l'une quelconque des revendications 1 à 3, dans lequel ce site de restriction unique est situé à une position comprise entre le nucléotide -1 et le nucléotide -10 de ce gène de la polyhédrine de baculovirus.

7. Procédé suivant l'une quelconque des revendications 1 à 4, dans lequel le gène de la polyhédrine de baculovirus est dérivé d'Autographa californica MNPV, de Trichoplusia ni MNPV, de Rachiplusia ou MNPV ou de Galleria mellonella MNPV.

8. Procédé suivant l'une quelconque des revendications 1 à 4, dans lequel le gène de baculovirus est dérivé d'Autographa californica MNPV.

9. Procédé suivant la revendication 4, dans lequel le gène hétérologue sélectionné est inséré au niveau du site Bam HI.

10. Procédé suivant l'une quelconque des revendications 1 à 9 pour la préparation de vecteurs de transfert désignés sous les noms de NRRL B-15428 ou de NRRL B-15778 lors du dépôt à la Agricultural Research Culture Collection.

**Patentansprüche**

**Patentansprüche für folgende Vertragsstaaten : BE, CH, DE, DK, ES, FR, GB, GR, IT, LI, LU, NL, SE**

1. Verfahren zur Herstellung eines rekombinanten Baculovirus-Expressionsvektors, der ein ausgewähltes, nicht-fusioniertes, rekombinantes, heterologes Gen in einer Wirtsinsektenzelle exprimieren kann, dadurch gekennzeichnet, daß es:

(a) die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;

(b) das Insertieren des Baculovirus-DNA-Fragments in ein Klonierungs-Vehikel zur Bildung eines Baculovirus-Gen-Transfervektors;

(c) die Erzeugung einer speziellen Restriktionsstelle zum Klonieren eines ausgewählten heterologen Gens, wobei die spezielle Restriktionsstelle innerhalb des Polyhedrin-Gens lokalisiert ist;

(d) die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist;

(e) die Herstellung eines rekombinanten Baculovirus-Transfervektors durch Insertieren von mindestens einem ausgewählten, heterologen Gen in die spezielle Restriktionsstelle, so daß sich das ausgewählte Gen unter der Transkriptions-Kontrolle des Baculovirus-Polyhedrin-Promotors befindet;

(f) das In-Berührung-Bringen des rekombinanten Baculovirus-Transfervektors mit Baculovirus-DNA, um die Rekombination zu bewirken und auf diese Weise ein Gemisch von rekombinanten und nicht-rekombinanten Baculoviren herzustellen; und

(g) das Isolieren eines rekombinanten Baculovirus-Expressionsvektors aus dem Gemisch

umfaßt.

2. Verfahren zur Herstellung eines rekombinanten Baculovirus-Transfervektors, der mindestens ein ausgewähltes heterologes Gen aufweist, dadurch gekennzeichnet, daß es

(a) die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;

(b) das Insertieren des Baculovirus-DNA-Fragments in ein Klonierungs-Vehikel zur Bildung eines Baculovirus-Gen-Transfervektors;

(c) die Erzeugung einer speziellen Restriktionsstelle zum Klonieren eines ausgewählten heterologen Gens, wobei die spezielle Restriktionsstelle innerhalb des Polyhedrin-Gens lokalisiert ist;

(d) die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist; und

(e) das Insertieren von mindestens einem ausgewählten, heterologen Gen in die spezielle Restriktionsstelle, so daß sich das ausgewählte Gen unter der Transkriptions-Kontrolle des Baculovirus-Polyhedrin-Promotors befindet,

umfaßt.

3. Verfahren zur Herstellung eines Baculovirus-Transfervektors, der eine spezielle Restriktionsstelle unter der Transkriptions-Kontrolle des Polyhedrin-Promotors aufweist, dadurch gekennzeichnet, daß es:

- die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;

- das Insertieren des DNA-Fragments in ein Klonierungs-Vehikel, um einen Baculovirus-Gen-Transfervektor herzustellen;

- die Erzeugung einer speziellen Restriktionsstelle zum Klonieren eines ausgewählten heterologen Gens, wobei die spezielle Restriktionsstelle innerhalb des Polyhedrin-Gens lokalisiert ist; und

- die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist,

umfaßt.

4. Verfahren zur Herstellung eines ausgewählten, nicht fusionierten, heterologen Polypeptids, dadurch gekennzeichnet, daß es das Infizieren einer empfindlichen Wirtszelle mit einem rekombinanten Baculovirus-Expresionsvektor umfaßt, der mindestens ein ausgewähltes, heterologes Gen, insertiert in ein Baculovirus-Polyhedrin-Gen zwischen das Polyhedrin-Transkriptions-Start-Signal und die Polyhedrin-ATG-Translations-Initiations-Start-Stelle, umfaßt sowie sich unter der Transkriptions-Kontrolle eines ausreichende Homologie mit einer in Fig. 3 gezeigten Polynukleotidsequenz aufweisenden Baculovirus-Polyhedrin-Promotors befindet.

5. Verfahren gemäß einem jeden der Ansprüche 1 bis 3, bei dem die spezielle Restriktionsstelle eine BamHI-Stelle ist.

6. Verfahren gemäß einem jeden der Ansprüche 1 bis 3, bei dem die spezielle Restriktionsstelle an einer Stelle im Bereich vom Nukleotid -1 zum Nukleotid -10 des Baculovirus-Polyhedrin-Gens lokalisiert ist.

7. Verfahren gemäß einem jeden der Ansprüche 1 bis 4, wobei das Baculovirus-Polyhedrin-Gen von Autographa californica MNPV, Trichoplusia ni MNPV, Rachiplusia ou MNPV oder Galleria mellonella MNPV abgeleitet ist.

8. Verfahren gemäß einem jeden der Ansprüche 1 bis 4, bei dem das Baculovirus-Gen von Autographa californica MNPV abgeleitet ist.

9. Verfahren gemäß Anspruch 4, bei dem das ausgewählte, heterologe Gen an einer BamHI-Stelle eingesetzt wird.

10. Rekombinanter Baculovirus-Expressionsvektor, der ein ausgewähltes, heterologes Gen in einer Wirtsinsektenzelle exprimieren kann, dadurch gekennzeichnet, daß er mindestens ein ausgewähltes, heterologes Gen umfaßt, das in ein Baculovirus-Polyhedrin-Gen an einer Stelle im Bereich von dem Polyhedrin-Transkriptions-Start-Signal zu der Polyhedrin-ATG-Translations-Initiations-Start-Stelle eingesetzt ist und sich unter der Transkriptions-Kontrolle eines ausreichende Homologie mit einer in Fig. 3 gezeigten Nukleotid-Sequenz aufweisenden Baculovirus-Polyhedrin-Promotors befindet.

11. Rekombinanter Transfervektor, der zum Einführen eines ausgewählten, heterologen Gens in ein Baculovirus-Genom befähigt ist, dadurch gekennzeichnet, daß er ein Klonierungs-Vehikel umfaßt, das eine DNA-Sequenz aufweist, die ein Baculovirus-Polyhedrin-Gen und mindestens ein ausgewähltes, heterologes Gen, das mit dem Baculovirus-Polyhedrin-Gen an einer Stelle im Bereich von dem Polyhedrin-Transkriptions-Start-Signal zu der Polyhedrin-ATG-Translations-Initiations-Start-Stelle verknüpft ist und sich unter der Transkriptions-Kontrolle eines Promotors des Baculovirus-Polyhedrin-Gens befindet, umfaßt, wobei der Baculovirus-Polyhedrin-Promotor eine ausreichende Homologie mit einer in Fig. 3 gezeigten Nukleotid-Sequenz und ausreichende flankierende Baculovirus-DNA-Sequenzen zur Erleichterung der homologen Rekombination aufweist.

12. Baculovirus-Transfervektor, dadurch gekennzeichnet, daß er einen ausreichende Homologie mit einer in Fig. 3 gezeigten Nukleotid-Sequenz aufweisenden Baculovirus-Polyhedrin-Promotor, ausreichende flankierende Baculovirus-DNA-Sequenzen zur Erleichterung der homologen Rekombination und eine spezielle Restriktionsstelle zum Klonieren eines ausgewählten, heterologen Gens aufweist, wobei die spezielle Restriktionsstelle in dem Polyhedrin-Gen an einer Stelle im Bereich von dem Polyhedrin-Transkriptions-Start-Signal zu der Polyhedrin-ATG-Translations-Initiations-Start-Stelle lokalisiert ist.

13. Rekombinanter Baculovirus-Expressionsvektor, der zur Herstellung eines ausgewählten, nicht-fusionierten, heterologen Proteins in einer Wirtsinsektenzelle befähigt ist, wobei der Expressionsvektor ein Baculovirus-Genom ist, das ein ausgewähltes, sein eigenes ATG-Signal einschließendes heterologes Gen unter der Transkriptionskontrolle eines ausreichende Homologie mit einer in Fig. 3 gezeigten Nukleotid-Sequenz aufweisenden Baculovirus-Polyhedrin-Promotors umfaßt.

14. Vektor gemäß einem jeden der Ansprüche 10 bis 13, bei dem der Baculovirus-Promotor von Autographa californica MNPV, Trichoplusia ni MNPV, Rachiplusia ou MNPV oder Galleria mellonella MNPV abgeleitet ist.

15. Vektor gemäß einem jeden der Ansprüche 10 bis 13, bei dem der Baculovirus-Promotor von Autographa californica MNPV abgeleitet ist.

**16.** Vektor gemäß einem jeden der Ansprüche 10 bis 13, bei dem das ausgewählte, heterologe Gen an einer BamHI-Stelle eingesetzt ist.

**17.** Vektor gemäß Anspruch 12, bei dem die spezielle Restriktionsstelle eine BamHI-Stelle ist.

**18.** Vektor gemäß einem jeden der Ansprüche 10 oder 11, bei dem das ausgewählte, heterologe Gen an einer Stelle im Bereich von dem Nukleotid -1 zu dem Nukleotid -10 des Baculovirus-Polyhedrin-Gens eingefügt ist.

**19.** Vektor gemäß Anspruch 12, bei dem die spezielle Restriktionsstelle an einer Stelle im Bereich von dem Nukleotid -1 zu dem Nukleotid -10 des Baculovirus-Polyhedrin-Gens lokalisiert ist.

**20.** Rekombinanter Baculovirus-Expressionsvektor von Anspruch 13, bei dem die gesamte oder ein Teil der DNA-Sequenz, die das Baculovirus-Polyhedrin-Struktur-Gen unter der Transkriptions-Kontrolle des Promotors kodiert, entfernt ist.

**21.** Baculovirus-Transfervektor von Anspruch 12, der als NRRL B-15428 oder NRRL B-15778, hinterlegt bei der Agricultural Research Culture Collection, bezeichnet ist.

**Patentansprüche für folgenden Vertragsstaat : AT**

**1.** Verfahren zur Herstellung eines rekombinanten Baculovirus-Expressionsvektors, der ein ausgewähltes, nicht-fusioniertes, rekombinantes, heterologes Gen in einer Wirtsinsektenzelle exprimieren kann, dadurch gekennzeichnet, daß es:

(a) die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;

(b) das Insertieren des Baculovirus-DNA-Fragments in ein Klonierungs-Vehikel zur Bildung eines Baculovirus-Gen-Transfervektors;

(c) die Erzeugung einer speziellen Restriktionsstelle zum Klonieren eines ausgewählten heterologen Gens, wobei die spezielle Restriktionsstelle innerhalb des Polyhedrin-Gens lokalisiert ist;

(d) die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist;

(e) die Herstellung eines rekombinanten Baculovirus-Transfervektors durch Insertieren von mindestens einem ausgewählten, heterologen Gen in die spezielle Restriktionsstelle, so daß sich das ausgewählte Gen unter der Transkriptions-Kontrolle des Baculovirus-Polyhedrin-Promotors befindet;

(f) das In-Berührung-Bringen des rekombinanten Baculovirus-Transfervektors mit Baculovirus-DNA, um die Rekombination zu bewirken und auf diese Weise ein Gemisch von rekombinanten und nicht-rekombinanten Baculoviren herzustellen; und

(g) das Isolieren eines rekombinanten Baculovirus-Expressionsvektors aus dem Gemisch

umfaßt.

**2.** Verfahren zur Herstellung eines rekombinanten Baculovirus-Transfervektors, der mindestens ein ausgewähltes heterologes Gen aufweist, dadurch gekennzeichnet, daß es

(a) die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;

(b) das Insertieren des Baculovirus-DNA-Fragments in ein Klonierungs-Vehikel zur Bildung eines Baculovirus-Gen-Transfervektors;

(c) die Erzeugung einer speziellen Restriktionsstelle zum Klonieren eines ausgewählten heterologen Gens, wobei die spezielle Restriktionsstelle innerhalb des Polyhedrin-Gens lokalisiert ist;

(d) die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist; und

(e) das Insertieren von mindestens einem ausgewählten, heterologen Gen in die spezielle Restriktionsstelle, so daß sich das ausgewählte Gen unter der Transkriptions-Kontrolle des Baculovirus-Polyhedrin-Promotors befindet,
umfaßt.

3. Verfahren zur Herstellung eines Baculovirus-Transfervektors, der eine spezielle Restriktionsstelle unter der Transkriptions-Kontrolle des Polyhedrin-Promotors aufweist, dadurch gekennzeichnet, daß es:
- die Spaltung von Baculovirus-DNA zur Herstellung eines DNA-Fragments, das ein mit einer in Fig. 3 gezeigten Nukleotid-Sequenz ausreichende Homologie aufweisendes Baculovirus-Polyhedrin-Gen und ausreichende, flankierende Baculovirus-DNA-Sequenzen zur Erleichterung der homologen Rekombination umfaßt;
- das Insertieren des DNA-Fragments in ein Klonierungs-Vehikel, um einen Baculovirus-Gen-Transfervektor herzustellen;
- die Erzeugung einer speziellen Restriktionsstelle zum Klonieren eines ausgewählten heterologen Gens, wobei die spezielle Restriktionsstelle innerhalb des Polyhedrin-Gens lokalisiert ist; und
- die Entfernung aller die Polyhedrin-Protein-Synthese kodierender Sequenzen aus dem Baculovirus-DNA-Fragment und gegebenenfalls das Entfernen einiger Sequenzen aus dem Baculovirus-DNA-Fragment stromaufwärts der Polyhedrin-ATG-Translations-Initiations-Start-Stelle, so daß die spezielle Restriktionsstelle in dem Polyhedrin-Gen an einer Stelle im Bereich von dem Polyhedrin-Transkriptions-Startsignal zu der ATG-Translations-Initiations-Start-Stelle lokalisiert ist,
umfaßt.

4. Verfahren zur Herstellung eines ausgewählten, nicht fusionierten, heterologen Polypeptids, dadurch gekennzeichnet, daß es das Infizieren einer empfindlichen Wirtszelle mit einem rekombinanten Baculovirus-Expressionsvektor umfaßt, der mindestens ein ausgewähltes, heterologes Gen, insertiert in ein Baculovirus-Polyhedrin-Gen zwischen das Polyhedrin-Transkriptions-Start-Signal und die Polyhedrin-ATG-Translations-Initiations-Start-Stelle, umfaßt sowie sich unter der Transkriptions-Kontrolle eines ausreichende Homologie mit einer in Fig. 3 gezeigten Polynukleotidsequenz aufweisenden Baculovirus-Polyhedrin-Promotors befindet.

5. Verfahren gemäß einem jeden der Ansprüche 1 bis 3, bei dem die spezielle Restriktionsstelle eine BamHI-Stelle ist.

6. Verfahren gemäß einem jeden der Ansprüche 1 bis 3, bei dem die spezielle Restriktionsstelle an einer Stelle im Bereich vom Nukleotid -1 zum Nukleotid -10 des Baculovirus-Polyhedrin-Gens lokalisiert ist.

7. Verfahren gemäß einem jeden der Ansprüche 1 bis 4, wobei das Baculovirus-Polyhedrin-Gen von Autographa californica MNPV, Trichoplusia ni MNPV, Rachiplusia ou MNPV oder Galleria mellonella MNPV abgeleitet ist.

8. Verfahren gemäß einem jeden der Ansprüche 1 bis 4, bei dem das Baculovirus-Gen von Autographa californica MNPV abgeleitet ist.

9. Verfahren gemäß Anspruch 4, bei dem das ausgewählte, heterologe Gen an einer BamHI-Stelle eingesetzt wird.

10. Verfahren gemäß einem jeden der Ansprüche 1 bis 9 zur Herstellung von als NRRL B-15428 oder NRRL B-15778 unter Hinterlegung bei der Agricultural Research Culture Collection bezeichneten Transfervektoren.

Fig.1

SHUTTLE VECTORS (MODIFIED)
(pAc 311, pAc 360, pAc 380)

_Fig. 2_

## Fig. 3

GGT CTG CGA GCA GTT GTT TGT TGT TAA AAATAA CAG CCATTG TAATGA GACG CACAAACT AAT AAT CAC AAACTG GAAAATGTC [TAT CAATA T] ATA GTT
-198                                            -142        -128             -110

pAc 380

pAc 373

GCT GAT ATC ATG GAG A [TA ATT AAA AT] GATA ACC ATC TC GC [AAA] TAAAT AAGTAT TTT ACT GTT TTC GTA ACAGT T TTG TAA TAA AAA AACC TA TAA ATA
-99 EcoRV                  -78                       -58

|  | pro | asp | tyr | ser | tyr | arg | pro | thr | ile | gly | arg | thr | tyr | val | tyr | asp | asn | lys | tyr | tyr | lys | asn | leu | gly |
|---|---|---|---|---|---|---|---|---|---|---|---|---|---|---|---|---|---|---|---|---|---|---|---|---|
| ATG | CCG | GAT | TAT | TCA | TAC | CGT | CCC | ACC | ATC | GGG | CGT | ACC | TAC | GTG | TAC | GAC | AAC | AAG | TAC | TAC | AAA | AAT | TTA | GGT |

+1 Msp I

pAc 360 —— pAc 311

| ala | val | ile | lys | asn | ala | lys | arg | lys | lys | his | phe | ala | glu | his | glu | ile | glu | glu | ala | thr | leu | asp | pro | leu |
|---|---|---|---|---|---|---|---|---|---|---|---|---|---|---|---|---|---|---|---|---|---|---|---|---|
| GCC | GTT | ATC | AAG | AAC | GCT | AAG | CGC | AAG | AAG | CAC | TTC | GCC | GAA | CAT | GAG | ATC | GAA | GAG | GCT | ACC | CTC | GAC | CCC | CTA |

76                                       Sau 3A          Taq I

Taq I

pAc 101

| asp | asn | tyr | leu | val | ala | glu | asp | pro | phe | leu | gly | pro | gly | lys | asn | gln | lys | leu | thr | leu | phe | lys | glu | ile |
|---|---|---|---|---|---|---|---|---|---|---|---|---|---|---|---|---|---|---|---|---|---|---|---|---|
| GAC | AAC | TAC | CTA | GTG | GCT | GAG | GAT | CCT | TTC | CTG | GGA | CCC | GGC | AAG | AAC | CAA | AAA | CTC | ACT | CTC | TTC | AAG | GAA | ATC |

151                             BamH I                 Msp I

Sau 3A

| arg | asn | val | lys | pro | asp | thr | met | lys | leu | val | val | gly | trp | lys | gly | lys | glu | phe | tyr | arg | glu | thr | trp | thr |
|---|---|---|---|---|---|---|---|---|---|---|---|---|---|---|---|---|---|---|---|---|---|---|---|---|
| CGT | AAT | GTT | AAA | CCC | GAC | ACG | ATG | AAG | CTT | GTC | GTT | GGA | TGG | AAA | GGA | AAA | GAG | TTC | TAC | AGG | GAA | ACT | TGG | ACC |

226                         Hind III

EP 0 127 839 B1

*Fig. 4*

AUTOGRAPHA CALIFORNICA
MNPV-IFN-β RECOMBINANT

AcMNPV, E2 Strain

1.) PURIFY
VIRAL DNA

pAc380
IFN-β

EcoRI

Hind III

IFN-β

1) Ca⁺⁺ COPRECIPITATE
2) TRANSFECT S FRUGIPERDA CELLS
3) PLAQUE PURIFY PROGENY VIRUS
4) SCREEN PLAQUE FOR THE
   OCCLUSION NEGATIVE PHENOTYPE

RECOMBINANT
VIRUS

1.) PLAQUE
    PURIFICATION

Fig. 5

Ac 380-IFN-β

1.) INFECT SPODOPTERA
    FRUGIPERDA CELLS

HUMAN INTERFERON-β
SECRETED

*Fig. 6*